# EUROPEAN PATENT SPECIFICATION

(11) **EP 0 973 913 B1**
(45) Date of publication and mention of the grant of the patent: **10.08.2005**
(21) Application number: 98918023.7
(22) Date of filing: 07.04.1998
(51) Int. Cl.: C12N 15/52, C12N 15/82, C12N 15/11, C12N 9/90, A01H 5/00

(54) **SOYBEAN PLANT PRODUCING SEEDS WITH REDUCED LEVELS OF RAFFINOSE SACCHARIDES AND PHYTIC ACID**
SOJABOHNENPFLANZEN, WELCHE SAMEN MIT VERINGERTEM GEHALT AN RAFFINOSE SACCHARIDEN UND PHYTINSÄURE PRODUZIEREN
PLANTE DE SOJA FOURNISSANT DES AMANDES A TENEURS REDUITES EN SACCHARIDES DE RAFFINOSE ET EN ACIDE PHYTIQUE

(30) Priority: 08.04.1997 US 835751
(43) Date of publication of application: 26.01.2000
(73) Proprietor: E.I. DU PONT DE NEMOURS AND COMPANY, Wilmington, Delaware 19898 (US)
(72) Inventor: HITZ, William, Dean, Wilmington, DE 19807 (US); SEBASTIAN, Scott, Anthony, Hockessin, DE (US)
(74) Representative: Grant, Anne Rosemary
(86) International application number: PCT/US1998/006822
(87) International publication number: WO 1998/045448

(56) References cited:
- WO-A-93/02196
- WO-A-93/07742
- WO-A1-97/37547
- DEAN-JOHNSON, MARGARET ET AL: "Biosynthesis of inositol in yeast. Primary structure of myo-inositol-1-phosphate synthase (EC 5.5.1.4) and functional analysis of its structural gene, the INO1 locus" J. BIOL. CHEM. (1989), 264(2), 1274-83 CODEN: JBCHA3;ISSN: 0021-9258, XP002070761
- JOHNSON, MARGARET DEAN ET AL: "1L-myo-Inositol 1-phosphate synthase from Arabidopsis thaliana" PLANT PHYSIOL. (1995), 107(2), 613-19 CODEN: PLPHAY;ISSN: 0032-0889, XP002070762 cited in the application

## Description

### FIELD OF THE INVENTION

This invention is in the field of plant molecular biology and genetics. More specifically, this invention pertains to soybean protein and meal products having significantly lower contents of raffinose, stachyose and phytic acid and significantly higher contents of sucrose and inorganic phosphate as a function of the use of soybean lines having a heritable, decreased capacity for the production of *myo*-inositol 1-phosphate in their seeds.

### BACKGROUND OF THE INVENTION

Raffinose saccharides are a group of D-galactose-containing oligosaccharides of sucrose that are widely distributed in plants. Raffinose saccharides are characterized by having the general formula O-α-D-galactopyranosyl-(1→6)ₙ-α-D-glucopyranosyl-(1→2)-β-D-fructofuranoside where n=1 through n=4 are known respectively as raffinose, stachyose, verbascose, and ajugose. In soybean seeds, raffinose and stachyose are the raffinose saccharides that are present in greatest quantity. In contrast, verbascose and ajugose are minor components and are generally not detected by standard analytical methods.

Extensive botanical surveys of the occurrence of raffinose saccharides have been reported in the scientific literature [Dey, P. M. In *Biochemistry of Storage Carbohydrates in Green Plants,* Academic Press, London, (1985) pp 53-129]. Raffinose saccharides are thought to be second only to sucrose among the nonstructural carbohydrates with respect to abundance in the plant kingdom. In fact, raffinose saccharides may be ubiquitous, at least among higher plants. Raffinose saccharides accumulate in significant quantities in the edible portion of many economically significant crop species. Examples include soybean *(Glycine max L.* Merrill), sugar beet *(Beta vulgaris*), cotton *(Gossypium hirsutum L.),* canola *(Brassica sp.)* and all of the major edible leguminous crops including beans (*Phaseolus sp*.), chick pea *(Cicer arietinum*), cowpea *(Vigna unguiculata*), mung bean *(Vigna radiata*), peas *(Pisum sativum),* lentil *(Lens culinaris)* and lupine *(Lupinus sp.).*

Although abundant in many species, raffinose saccharides are an obstacle to the efficient utilization of some economically important crop species. Raffinose saccharides are not digested directly by animals, primarily because α-galactosidase is not present in the intestinal mucosa [Gitzelmann and Auricchio.(1965) *Pediatrics 36*:231-236; Rutloff et al. (1967) *Nahrung. 11*:39-46]. However, microflora in the lower gut are readily able to ferment the raffinose saccharides which results in an acidification of the gut and production of carbon dioxide, methane and hydrogen [Murphy et al. (1972) *J. Agr. Food Chem. 20*:813-817; Cristofaro et al. In *Sugars in* *Nutrition*, (1974) Chapter 20, 313-335; Reddy et al. (1980) *J. Food Science 45*:1161-1164]. The resulting flatulence can severely limit the use of leguminous plants in animal, including human, diets. It is unfortunate that the presence of raffinose saccharides restricts the use of soybeans in animal, including human, diets because otherwise this species is an excellent source of protein and fiber.

The soybean is well-adapted to machinery and facilities for harvesting, storing and processing that are widely available in many parts of the world. In the U.S. alone, approximately 28 million metric tons of meal were produced in 1988 *[Oil Crops Situation and Outlook Report,* Apr. 1989, U.S. Dept. of Agriculture, Economic Research Service]. Typically, hulls are removed and then the oil is extracted with hexane in one of several extraction systems. The remaining defatted flakes can then be used for a variety of commercial soy protein products [*Soy Protein Products, Characteristics, Nutritional Aspects and Utilization* (1987) Soy Protein Council]. Foremost among these in volume of use is soybean meal, the principle source of protein in diets used for animal feed, especially those for monogastric animals such as poultry and swine.

Although the soybean is an excellent source of vegetable protein, there are inefficiencies associated with its use that appear to be due to the presence of raffinose saccharides. Compared to maize, the other primary ingredient in animal diets, gross energy utilization for soybean meal is low [Potter and Potchanakom. In: *Proceedings World Soybean Conference III*, (1984) 218-224]. For example, although soybean meal contains approximately 6% more gross energy than ground yellow corn, it has about 40 to 50% less metabolizable energy when fed to chickens. This inefficiency of gross energy utilization does not appear to be due to problems in digestion of the protein fraction of the meal, but rather due to the poor digestion of the carbohydrate portion of the meal. It has been reported that removal of raffinose saccharides from soybean meal by ethanol extraction results in a large increase in the metabolizable energy for broilers [Coon, C. N. et al. In: *Proceedings Soybean Utilization Alternatives,* University of Minnesota, (1988) 203-211]. Removal of the raffinose saccharides was associated with increased utilization of the cellulosic and hemicellulosic fractions of the soybean meal.

A variety of processed vegetable protein products are produced from soybean. These range from minimally processed, defatted items such as soybean meal, grits, and flours to more highly processed items such as soy protein concentrates and soy protein isolates. In other soy protein products the oil is not extracted, full-fat soy flour for example. In addition to these processed products, there are also a number of speciality products based on traditional Oriental processes, which utilize the entire bean as the starting material. Examples include soy milk, soy sauce, tofu, natto, miso, tempeh and yuba.

Examples of use of soy protein products in human foods include soy protein concentrates, soy protein isolates, textured soy protein, soy milk and infant formula. Facilities and methods to produce protein concentrates and isolates from soybeans are available across the world. For example in WO97/37547, a method for the manufacture of an isoflavone, enriched soy protein product was described. One of the problems faced by producers of soy protein concentrates and isolates is the challenge of selectively purifying the protein away from the raffinose saccharides. Considerable equipment and operating costs are incurred as a result of removing the large amounts of raffinose saccharides that are present in soybeans.

The problems and costs associated with raffinose saccharides could be reduced or eliminated through the availability of genes that confer a reduction of raffinose saccharide content of soybean seeds. Such genes could be used to develop soybean varieties having inherently reduced raffinose saccharide content. Soybean varieties with inherently reduced raffinose saccharide content would improve the nutritional quality of derived soy protein products and reduce processing costs associated with the removal of raffinose saccharides. Low raffinose saccharide soybean varieties would be more valuable than conventional varieties for animal and human diets and would allow mankind to more fully utilize the desirable nutritional qualities of this edible legume.

*myo*-Inositol hexaphosphate (also know as "phytic acid") and raffinose saccharides share *myo*-inositol as a common intermediate in their synthesis [Ishitani, M. et al., (1996) *The Plant Journal 9:537-548*]. Like raffinose saccharides, phytic acid is a nearly ubiquitous component of angiosperm seeds [Raboy, V. In: *Inositol Metabolism in Plants* (1990) Wiley-Liss, New York, pp 55-76]. While phytic acid typically accounts for 50 to 70% of the total phosphate in seeds such as soybean and corn, that phosphate is only poorly available to mono-gastric animals. In addition to being only partially digestible, the presence of phytic acid in animal rations leads to excretion of other limiting nutrients such as essential amino acids, calcium and zinc [Mroz, Z. et al., (1994) *J. Animal Sci. 72:*126-132; Fox et al., In: *Nutritional Toxicology Vol. 3,* Academic Press, San Diego (1989) pp. 59-96]. Since soybean meal is a major portion of many animal feed rations, a meal with decreased amounts of phytic acid along with increased amounts of available phosphate should lead to improved feed efficiency in soy containing rations. Indeed enzymatic treatment of soybean meal containing rations to partially hydrolyze the phosphate groups from phytic acid improves both phosphate availability and the availability of other limiting nutrients [Mroz et al., *supra*; Pen et al., (1993) *Biotechnology 11:811-814*].

Surveys of commercial and wild soybean germplasm indicate that limited genetic variability for seed phytic acid content exists [Raboy et al., (1984) *Crop Science 24:431-434*]. In light of these factors, it is apparent that soybean plants with heritable, substantially reduced levels of raffinose saccharides and phytic acid in their seeds are needed.

### SUMMARY OF THE INVENTION

The instant invention pertains to a soybean plant with a heritable phenotype of (i) a seed phytic acid content of less than 17 µmol/g, (ii) a seed content of raffinose plus stachyose combined of less than 14.5 µmol/g, and (iii) a seed sucrose content of greater than 200 µmol/g, the phenotype due to a decreased capacity for the synthesis of *myo*-inositol 1-phosphate in the seeds of the plant. The invention also pertains to seeds derived from this plant.

More specifically, this invention pertains to an isolated nucleic acid fragment encoding a soybean *myo*-inositol 1-phosphate synthase or its complement, and a chimeric gene comprising this nucleic acid fragment or a subfragment of this nucleic acid fragment, operably linked to suitable regulatory sequences, wherein expression of the chimeric gene results in a decrease in expression of a native gene encoding a soybean *myo*-inositol 1-phosphate synthase.

Another embodiment of the instant invention is an isolated nucleic acid fragment encoding a mutant myo-inositol 1-phosphate synthase having decreased capacity for the synthesis of *myo*-inositol-1-phospate.

Yet another embodiment of the instant invention is a soybean plant having in its genome a chimeric gene comprising a nucleic acid fragment encoding a soybean *myo*-inositol 1-phosphate synthase or the complement of the nucleic acid fragment, operably linked to suitable regulatory sequences, wherein expression of the chimeric gene results in a decrease in expression of a native gene encoding a soybean *myo*-inositol-1-phosphate synthase.

Still another embodiment of the instant invention is a soybean plant homozygous for at least one gene encoding a mutant *myo*-inositol 1-phosphate synthase having decreased capacity for the synthesis of *myo*-inositol 1-phosphate, the gene conferring a heritable phenotype of (i) a seed phytic acid content of less than 17 µmol/g, (ii) a seed content of raffinose plus stachyose combined of less than 14.5 µmol/g, and (iii) a seed sucrose content of greater than 200 µmol/g.

The instant invention also pertains to seeds and protein products derived from the seeds of any of the plants described above.

Yet another embodiment of the instant invention is a method for making a soybean plant with a heritable phenotype of (i) a seed phytic acid content less than 17 µmol/g, (ii) a seed content of raffinose plus stachyose combined of less than 14.5 µmol/g, and (iii) a seed sucrose content of greater than 200 µmol/g, the method comprising (a) crossing the soybean line LR33 or any soybean plant having in its genome a chimeric gene comprising a nucleic acid fragment encoding a soybean *myo*-inositol 1-phosphate synthase or the complement of the nucleic acid fragment, operably linked to suitable regulatory sequences, or a soybean plant homozygous for at least one gene encoding a mutant *myo*-inositol 1-phosphate synthase having decreased capacity for the synthesis of *myo*-inositol 1-phosphate, with an elite soybean plant; and (b) selecting progeny plants of the cross of step (a) that have a heritable phenotype of (i) a seed phytic acid content less than 17 µmol/g, (ii) a seed content of raffinose plus stachyose combined of less than 14.5 µmol/g, and (iii) a seed sucrose content of greater than 200 µmol/g.

The instant invention also embodies a method for producing a soy protein product comprising (a) crossing an agronomically elite soybean plant with LR33 or any of the soybean plants described above; (b) screening the seed of progeny plants obtained from step (a) for (i) a seed phytic acid content less than 17 µmol/g, (ii) a seed content of raffinose plus stachyose combined of less than 14.5 µmol/g, and (iii) a seed sucrose content of greater than 200 µmol/g; and (c) processing the seed selected in step (b) to obtain the desired soybean protein product.

Finally, the instant invention embodies a method of using a soybean plant homozygous for at least one gene encoding a mutant *myo*-inositol 1-phosphate synthase having decreased capacity for the synthesis of *myo*-inositol 1-phosphate, the gene conferring a heritable phenotype of (i) a seed phytic acid content less than 17 µmol/g, (ii) a seed content of raffinose plus stachyose combined of less than 14.5 µmol/g, and (iii) a seed sucrose content of greater than 200 µmol/g to produce progeny lines, the method comprising (a) crossing a soybean plant comprising a mutant *myo*-inositol 1-phosphate synthase having decreased capacity for the synthesis of *myo*-inositol 1-phosphate with any soybean parent which does not comprise the mutation, to yield a F 1 hybrid; (b) selfing the F 1 hybrid for at least one generation; and (c) identifying the progeny of step (b) homozygous for at least one gene encoding a mutant *myo*-inositol 1-phosphate synthase having decreased capacity for the synthesis of *myo*-inositol 1-phosphate, the gene conferring a heritable phenotype of (i) a seed phytic acid content less than 17 µmol/g, (ii) a seed content of raffinose plus stachyose combined of less than 14.5 µmol/g, and (iii) a seed sucrose content of greater than 200 µmol/g.

### BRIEF DESCRIPTION OF THE DRAWINGS AND SEQUENCE LISTINGS

The invention can be more fully understood from the figure and the Sequence Listings which form a part of this application. The Sequence Listings contain the three letter codes for amino acids as defined in 37 C.F.R. 1.822 which are incorporated herein by reference.

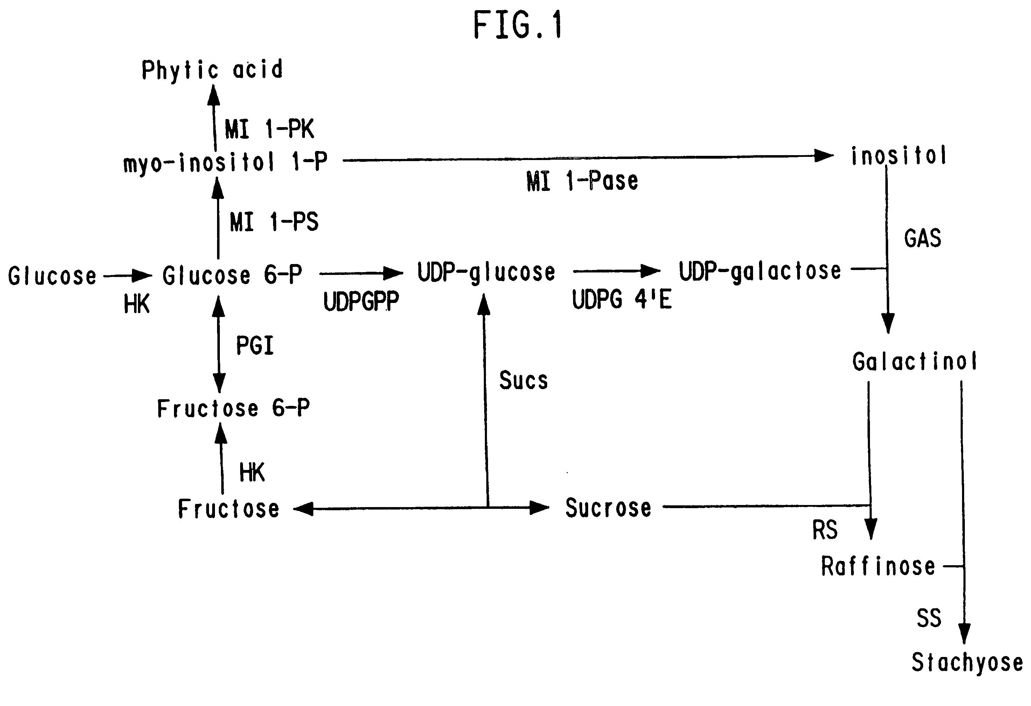

Figure 1. Glucose metabolism to phytic acid, raffinose and stachyose in soybean seeds. The common co-factors, ATP, ADP, UTP, UDP, pyrophosphave and inorganic phosphate (Pi) are not shown. The enzymes are listed by abbreviation: hexokinase (HK); phosphoglucoisomerase (PGI); UDPglucose pyrophosphorylase (UDPGPP); UDPglucose 4' epimerase (UDPG 4'E); *myo*-inositol 1-phosphate synthase (MI 1-PS); *myo*-inositol 1-phosphatase (MI 1-Pase); *myo*-inositol 1-phosphate kinase (MI 1-PK); galactinol synthase (GAS); sucrose synthase (SucS); raffinose synthase (RS); and stachyose synthase (SS).

SEQ ID NO:1 is the 5' to 3' nucleotide sequence of the 1782 bases of the cDNA encoding the wild type soybean *myo*-inositol 1-phosphate synthase present in clone p5bmi-lps.

SEQ ID NO:2 is the 510 amino acid sequence deduced from the open reading frame in SEQ ID NO:1.

SEQ ID NO:3 is the nucleotide sequence of the upstream (5') primer used in the isolation of the *myo*-inositol-1-phosphate synthase cDNA from soybean line LR33.

SEQ ID NO:4 is the nucleotide sequence of the downstream (3') primer used in the isolation of the *myo*-inositol 1-phosphate synthase cDNA from soybean line LR33.

SEQ ID NO:5 is the nucleotide sequence of the 1533 bases of cDNA encoding the LR33 *myo*-inositol 1-phosphate synthase present in clone LR33-10.

SEQ ID NO:6 is the 510 amino acid sequence deduced from the open reading frame in SEQ ID NO:5.

SEQ ID NO:7 is the nucleotide sequence of the upstream (5') primer used for PCR amplification of the wild type allele encoding *myo*-inositol 1-phosphate synthase from genomic DNA samples.

SEQ ID NO:8 is the nucleotide sequence of the upstream (5') primer used for PCR amplification of the LR33 allele encoding *myo*-inositol-1-phosphate synthase from genomic DNA samples.

### BIOLOGICAL DEPOSITS

The following biological materials have been deposited under the terms of the Budapest Treaty at American Type Culture Collection (ATCC), 12301 Parklawn Drive, Rockville, MD 20852, and bear the following accession numbers:

| Designation | Material | Accession Number | Date of Deposit |
|---|---|---|---|
| LR33 | Seed | ATCC 97988 | April 17, 1997 |
| 4E76 | Seed | ATCC 97971 | April 4, 1997 |
| p5bmi-1ps | Plasmid | ATCC 97970 | April 4, 1997 |

### DETAILED DESCRIPTION

In the context of this disclosure, a number of terms shall be utilized. As used herein, "soybean" refers to the species *Glycine max, Glycine soja*, or any species that is sexually cross compatible with *Glycine max*. A "line" is a group of plants of similar parentage that display little or no genetic variation between individuals for a least one trait. Such lines may be created by one or more generations of self-pollination and selection, or vegetative propagation from a single parent including by tissue or cell culture techniques. "Mutation" refers to a detectable and heritable genetic change (either spontaneous or induced) not caused by segregation or genetic recombination. "Mutant" refers to an individual, or lineage of individuals, possessing a mutation. A "population" is any group of individuals that share a common gene pool. In the instant invention, this includes M1, M2, M3, M4, F1, and F2 populations. As used herein, an "M 1 population" is the progeny of seeds (and resultant plants) that have been exposed to a mutagenic agent, while "M2 population" is the progeny of self-pollinated M1 plants, "M3 population" is the progeny of self-pollinated M2 plants, and "M4 population" is the progeny of self-pollinated M3 plants. As used herein, an "F1 population" is the progeny resulting from cross pollinating one line with another line. The format used herein to depict such a cross pollination is "female parent*male parent". An "F2 population" is the progeny of the self-pollinated F1 plants. An "F2-derived line" or "F2 line" is a line resulting from the self-pollination of an individual F2 plant. An F2-derived line can be propagated through subsequent generations (F3, F4, F5 etc.) by repeated self-pollination and bulking of seed from plants of said F2-derived line.

The term "nucleic acid" refers to a large molecule which can be single-stranded or double-stranded, composed of monomers (nucleotides) containing a sugar, a phosphate and either a purine or pyrimidine. A "nucleic acid fragment" is a fraction of a given nucleic acid molecule. A "subfragment" refers to a contiguous portion of a nucleic acid fragment comprising less than the entire nucleic acid fragment. "Complementary" refers to the specific pairing of purine and pyrimidine bases that comprise nucleic acids: adenine pairs with thymine and guanine pairs with cytosine. Thus, the "complement" of a first nucleic acid fragment refers to a second nucleic acid fragment whose sequence of nucleotides is complementary to the first nucleic acid sequence.

In higher plants, deoxyribonucleic acid (DNA) is the genetic material while ribonucleic acid (RNA) is involved in the transfer of the information in DNA into proteins. A "genome" is the entire body of genetic material contained in each cell of an organism. The term "nucleotide sequence" refers to the sequence of DNA or RNA polymers, which can be single- or double-stranded, optionally containing synthetic, non-natural or altered nucleotide bases capable of incorporation into DNA or RNA polymers. The term "oligomer" refers to short nucleotide sequences, usually up to 100 bases long. As used herein, the term "homologous to" refers to the relatedness between the nucleotide sequence of two nucleic acid molecules or between the amino acid sequences of two protein molecules. Estimates of such homology are provided by either DNA-DNA or DNA-RNA hybridization under conditions of stringency as is well understood by those skilled in the art (Hames and Higgins, Eds. (1985) Nucleic Acid Hybridisation, IRL Press, Oxford, U.K.); or by the comparison of sequence similarity between two nucleic acids or proteins, such as by the method of Needleman et al. (J. Mol. Biol. (1970) 48:443-453). As used herein, "substantially similar" refers to nucleotide sequences that have more than 90% overall identity at the nucleotide level with the coding region of the claimed sequence, such as genes and pseudo-genes corresponding to the coding regions. The nucleic acid fragments described herein include molecules which comprise possible variations, both man-made and natural, such as but not limited to (a) those that involve base changes that do not cause a change in an encoded amino acid, or (b) which involve base changes that alter an amino acid but do not affect the functional properties of the protein encoded by the DNA sequence, (c) those derived from deletions, rearrangements, amplifications, random or controlled mutagenesis of the nucleic acid fragment, and (d) even occasional nucleotide sequencing errors.

"Gene" refers to a nucleic acid fragment that expresses a specific protein, including regulatory sequences preceding (5' non-coding) and following (3' non-coding) the coding region. "Native" gene refers to an isolated gene with its own regulatory sequences as found in nature. "Chimeric gene" refers to a gene that comprises heterogeneous regulatory and coding sequences not found in nature. "Endogenous" gene refers to the native gene normally found in its natural location in the genome and is not isolated. A "foreign" gene refers to a gene not normally found in the host organism but that is introduced by gene transfer. "Pseudo-gene" refers to a genomic nucleotide sequence that does not encode a functional enzyme.

"Coding sequence" refers to a DNA sequence that codes for a specific protein and excludes the non-coding sequences. It may constitute an "uninterrupted coding sequence", i.e., lacking an intron or it may include one or more introns bounded by appropriate splice junctions. An "intron" is a nucleotide sequence that is transcribed in the primary transcript but that is removed through cleavage and re-ligation of the RNA within the cell to create the mature mRNA that can be translated into a protein.

"Initiation codon" and "termination codon" refer to a unit of three adjacent nucleotides in a coding sequence that specifies initiation and chain termination, respectively, of protein synthesis (mRNA translation). "Open reading frame" refers to the coding sequence uninterrupted by introns between initiation and termination codons that encodes an amino acid sequence.

"RNA transcript" refers to the product resulting from RNA polymerase-catalyzed transcription of a DNA sequence. When the RNA transcript is a perfect copy of the DNA sequence, it is referred to as the primary transcript or it may be a RNA sequence derived from posttranscriptional processing of the primary transcript and is then referred to as the mature RNA. "Messenger RNA (mRNA)" refers to the RNA that is without introns and that can be translated into protein by the cell. "cDNA" refers to a double-stranded DNA that is derived from mRNA. "Sense" RNA refers to an RNA transcript that includes the mRNA. "Antisense RNA" refers to an RNA transcript that is complementary to all or part of a target primary transcript or mRNA and that blocks the expression of a target gene by interfering with the processing, transport and/or translation of its primary transcript or mRNA. The complementarity of an antisense RNA may be with any part of the specific gene transcript, i.e., at the 5' non-coding sequence, 3' non-coding sequence, introns, or the coding sequence. In addition, as used herein, antisense RNA may contain regions of ribozyme sequences that increase the efficacy of antisense RNA to block gene expression. "Ribozyme" refers to a catalytic RNA and includes sequence-specific endoribonucleases.

As used herein, "suitable regulatory sequences" refer to nucleotide sequences in native or chimeric genes that are located upstream (5'), within, and/or downstream (3') to the nucleic acid fragments of the invention, which control the expression of the nucleic acid fragments of the invention.

"Promoter" refers to a DNA sequence in a gene, usually upstream (5') to its coding sequence, which controls the expression of the coding sequence by providing the recognition for RNA polymerase and other factors required for proper transcription. In artificial DNA constructs promoters can also be used to transcribe antisense RNA. Promoters may also contain DNA sequences that are involved in the binding of protein factors which control the effectiveness of transcription initiation in response to physiological or developmental conditions. It may also contain enhancer elements. An "enhancer" is a DNA sequence which can stimulate promoter activity. It may be an innate element of the promoter or a heterologous element inserted to enhance the level and/or tissue-specificity of a promoter. "Constitutive promoters" refers to those that direct gene expression in all tissues and at all times. "Tissue-specific" or "development-specific" promoters as referred to herein are those that direct gene expression almost exclusively in specific tissues, such as leaves or seeds, or at specific development stages in a tissue, such as in early or late embryogenesis, respectively. The term "expression", as used herein, refers to the transcription and stable accumulation of the sense (mRNA) or the antisense RNA derived from the nucleic acid fragment(s) of the invention that, in conjunction with the protein apparatus of the cell, results in altered levels of *myo-inositol 1-phosphate* synthase. "Antisense inhibition" refers to the production of antisense RNA transcripts capable of preventing the expression of the target protein. "Overexpression" refers to the production of a gene product in transgenic organisms that exceeds levels of production in normal or non-transformed organisms. "Cosuppression" refers to the expression of a foreign gene which has substantial homology to an endogenous gene resulting in the suppression of expression of both the foreign and the endogenous gene. "Altered levels" refers to the production of gene product(s) in transgenic organisms in amounts or proportions that differ from that of normal or non-transformed organisms. The present invention also relates to vectors which include nucleotide sequences of the present invention, host cells which are genetically engineered with vectors of the invention and the production of polypeptides of the invention by recombinant techniques. "Transformation" herein refers to the transfer of a foreign gene into the genome of a host organism and its genetically stable inheritance. "Fertile" refers to plants that are able to propagate sexually.

"Raffinose saccharides" refers to the family of oligosaccharides with the general formula O-α-D-galactopyranosyl-(1→6)ₙ-α-D-glucopyranosyl-(1→2)-β-D-fructofuranoside where n=1 to 4. In soybean seeds, the term refers more specifically to the members of the family containing one (raffinose) and two (stachyose) galactose residues. Although higher galactose polymers are known (e.g., verbascose and ajugose), the content of these higher polymers in soybean is below standard methods of detection and therefore do not contribute significantly to total raffinose saccharide content.

"Plants" refer to photosynthetic organisms, both eukaryotic and prokaryotic, whereas the term "Higher plants" refers to eukaryotic plants.

This invention provides a mutated form of a soybean gene and methods to improve the carbohydrate and phytic acid composition of soybean seeds and derived products. The invention teaches examples of a method to identify mutations in this gene and to use derived mutant soybean lines to reduce the raffinose saccharide and phytic acid content of soybean seeds. This invention also teaches methods of using gene silencing technology and the soybean gene sequence for *myo*-inositol-1-phosphate synthase discovered in the instant invention to reduce the raffinose saccharide content in soybean seeds.

Seeds derived from the plants of the present invention express an improved soluble carbohydrate content relative to commercial varieties. The improvements result in a reduced total raffinose plus stachyose content. The carbohydrate profile of these lines is dramatically different from the profiles seen in elite or germplasm lines used in or produced by other soybean breeding programs.

Two separate methods to produce the novel soybean genes of the present invention are taught. The first approach marks the first successful attempt to induce a mutation conferring low raffinose plus stachyose content. This approach resulted in the discovery of two major genes, one of which is described in detail in this invention, that can be used to develop soybean lines that are superior (in terms of reducing combined raffinose and stachyose content) to any lines previously reported. The second approach utilizes the gene sequences taught in this invention applied in transgenic methods of specific gene silencing to achieve results similar to those obtained through random mutagenesis and screening.

Applicants initially sought to introduce random mutations in the genome of wild-type soybeans by chemical mutagenesis. The instant invention employed NMU (N-nitroso-N-methylurea) as the mutagenic agent, although other agents known to alter DNA structure and sequence could have been used. Following treatment NMU, soybean seeds were sown for several generation and screened for the desired phenotype; of primary importance was the alteration of raffinose saccharide content. Initial screening of mutagenized soybean populations revealed two lines, LR33 and LR28, that appeared to be low in raffinose saccharides (LR28 is disclosed in World Patent Publication WO93/07742). The low raffinose saccharide phenotype of LR33 was demonstrated to be inheritable by analysis of three subsequent generations of LR33 produced by self-fertilization (Table 1).

The physiological defect in LR33 leading to the unique phenotype displayed by this line was identified and characterized by conducting a series of elegant genetic and biochemical studies (see Example 2, *infra*). The defect in LR33 was shown to be genetically and biochemically distinct from the mutation in LR28 that leads to the low stachyose phenotype of that line. Moreover, the mutation in LR33 demonstrates greater pleiotrophy than the defect in LR28; the instant specification demonstrates that the LR33 phenotype includes not only reduced raffinose saccharide content, but also results in alterations in seed phytic acid, inorganic phosphate and sucrose levels. Further analyses confirmed that genetic information derived from LR33 alone could confer this unique phenotype on progeny soybean lines, and that the mutant gene or genes in LR33 are not simply genetic modifiers that enhance the phenotypic expression of genes derived from other mutant soybean lines.

The specific biochemical defect responsible for the heritable phenotype demonstrated by LR33 and its progeny has been identified. This was accomplished by consideration of the biosynthesis of raffinose saccharides and the control of phytic acid and inorganic phosphate levels in soybean seeds. Based upon these known biosynthetic pathways, a series of biochemical studies and subsequent molecular genetic analyses identified defect in LR33 seeds as an alteration in *myo*-inositol 1-phosphate synthase activity, leading to a decreased capacity for synthesis of *myo*-inositol 1-phosphate.

The biosynthesis of raffinose and stachyose has been fairly well characterized [see Dey, P. M. In Biochemistry of Storage Carbohydrates in Green Plants (1985)]. *Myo*-Inositol hexaphosphate or phytic acid and raffinose saccharides share *myo*-inositol as a common intermediate in their synthesis [Ishitani,M et al. The Plant Journal (1996) 9:537-548]. Starting with glucose as a carbon source, the pathway describing the synthesis of phytic acid, raffinose and stachyose in maturing soybean seeds is shown in Figure I.

By the interconversions shown in Figure 1, either glucose or sucrose can be the starting material for the polyol portion of phytic acid, all of the hexoses that make up raffinose and stachyose and the re-cycled portion of the galactose donor to raffinose synthase and stacyhose synthase, *myo*-inositol. The end products of these interconversions that accumulate in mature, wild type soybean seeds are, in order of prominance by mass, sucrose, stachyose, phytic acid, and raffinose.

The committed reaction of raffinose saccharide biosynthesis involves the synthesis of galactinol (O-α-D-galactopyranosyl-(1→1)-*myo*-inositol) from UDPgalactose and *myo*-inositol. The enzyme that catalyzes this reaction is galactinol synthase. Synthesis of raffinose and higher homologues in the raffinose saccharide family from sucrose is catalyzed by the galactosyltransferases raffinose synthase and stachyose synthase.

Control over the ratio of these end products may be affected by altering the rate of conversion at many of the enzyme catalyzed steps in Figure 1. That control can be affected by altering enzyme expression level or by altering the intrinsic activity of the enzyme. The resulting mix of end products coming from the modified pathway may then comprise new proportions of the original end products as well as new product mixes which include accumulations of some of the normal intermediates. The exact mix and composition will depend upon both the enzyme which has been altered in its activity and the degree of that alteration.

The six enzymes *myo*-inositol 1-phosphate synthase, *myo*-inositol 1-phosphatase, UDP-glucose 4' epimerase, galactinol synthase, raffinose synthase, and stachyose synthase could be reduced in activity to decrease either raffinose or stachyose synthesis without decreasing sucrose content. Of these six, the three enzymes unique to raffinose and stachose synthesis could be decreased in activity without decreasing phytic acid content. Only *myo*-inositol 1-phosphate synthase appears to be involved in the synthesis of all three end products and may therefore change the amount of all three end products simultaneosly if its activity is decreased.

The instant invention teaches the ability to simultaneously reduce raffinose saccharide and phytic acid content and increase sucrose and inorganic phosphate content in soybean seeds by reducing *myo*-inositol 1-phosphate synthase activity in the cells of soybean seeds. The instant examples describe generation and discovery of a mutant form of this enzyme wherein a point mutation in the nucleotide sequence encoding this enzyme results in an amino acid substitution which, in turn, lowers intracellular enzymatic activity. It is well known to the skilled artisan that other mutations within the coding region for *myo*-inositol 1-phosphate synthase can result in decreased enzymatic activity and thus result in the instant seed phenotype. Using well known techniques of heterologous gene expression and *in vitro* mutagenesis, and employing the various enzymatic assays described herein, the skilled artisan could identify other mutations within the *myo*-inositol 1-phosphate synthase coding region that result in decreased enzymatic activity without undue experimentation. These mutated *myo*-inositol 1-phosphate synthase genes could then be introduced into the soybean genome (see U.S. Pat. No. 5,501,967) and result in new soybean varieties displaying the instant phenotype.

Alternatively, gene silencing techniques such as antisense inhibition technology (U.S. Pat. No. 5,107,065) and cosuppression (U.S. Pat. No. 5,231,020) may be employed to reduce the intracellular *myo*-inositol 1-phosphate synthase activity in the cells of soybean seeds. The instant specification teaches the sequence of the gene encoding the wild type soybean *myo*-inositol 1-phosphate synthase enzyme. The skilled artisan will readily appreciate how to make and how to use chimeric genes comprising all or part of the wild type sequence or substantially similar sequences to reduce *myo*-inositol 1-phosphate synthase activity in soybean seeds.

Accordingly, the instant invention pertains to the identity, characterization and manipulation of a soybean enzyme that results in the alteration of raffinose saccharide, sucrose, phytic acid and inorganic phosphate content of soybean seeds, thus leading to valuable and useful soybean products. As taught herein, reduction of *myo*-inositol 1-phosphate synthase enzymatic activity by any of several means will result in soybean seeds displaying the instant phenotype.

### EXAMPLES

The present invention is further defined in the following Examples, in which all parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain, and without departing from the spirit and scope thereof, can make various changes and modifications of the invention to adapt it to various usages and conditions. Further, the present invention is not to be limited in scope by the biological materials deposited, since the deposited materials are intended to provide illustrations of materials from which many embodiments may be derived. All such modifications are intended to fall within the scope of the appended claims.

Usual techniques of molecular biology such as bacterial transformation, agarose gel electrophoresis of nucleic acids and polyacrylamide electrophoresis of proteins are referred to by the common terms describing them. Details of the practice of these techniques, well known to those skilled in the art, are described in detail in [Sambrook, et al. (Molecular Cloning, A Laboratory Manual, 2nd ed. (1989), Cold Spring Harbor Laboratory Press]. Various solutions used in the experimental manipulations are referred to by their common names such as "SSC", "SSPE", "Denhardt's solution", etc. The composition of these solutions may be found by reference to Appendix B of Sambrook, et al. [*supra*].

### EXAMPLE I

### DISCOVERY OF A SOYBEAN GENE CONFERRING IMPROVED CARBOHYDRATE COMPOSITION

### Assays for Raffinose Saccharide Content

The following assays were used to analyze soybean seeds for raffinose saccharide content. Prior to each of the analytical measures for determination of raffinose saccharide content, seeds were allowed to air dry for at least one week to a moisture content of approximately 8% and then stored at approximately 40 to 50°C and 40% relative humidity. Inventors' own measurements of many such air-dried samples indicated that the moisture content did not vary significantly from 8% (range of 7 to 10%) when stored at these conditions.

For each individual raffinose saccharide assay, typically five to ten soybeans from a given plant were ground in a Cyclotech 1093 Sample Mill (Tecator, Box 70 S-26301, Hoganas, Sweden) equipped with a 100 mesh screen to yield a seed powder that was then analyzed. In most cases, raffinose saccharide content was determined for seed or derived products containing an "as is" moisture content of approximately 8%. In these cases, "as is" values were converted to a dry basis (db) by dividing measurements by 0.92. For comparison among certain lines or among certain soy protein products, the ground seed powder was placed in a forced air oven at 45°C until the samples reached constant weight (0% moisture) prior to analysis. Hence, all raffinose saccharide measurements reported within this specification are on a common dry basis unless otherwise specificied.

As describe below, three assays ("enzymatic", "TLC", and "HPLC") were used to determine raffinose saccharide content of soybean seeds. All three were performed on seed powder derived from the aforementioned grinding process.

In preparation for the "enzymatic" assay, after grinding each seed sample, approximately 30 mg of the resultant powder was weighed into a 13 x 100 mm screw cap tube and 1.6 mL of chloroform and 1.4 mL of methanol:water (4:3, v/v) was added. The precise powder weight of each sample was recorded and used to adjust the following assay results for sample to sample weight differences. The tubes were then capped, placed in racks and shaken on a rotary shaker for 60 min at 1800 rpm at room temperature. After extraction, the contents of the tubes were allowed to settle for 15 min. After settling, a 15 µL aliquot of the methanol:water phase was placed in a well of a 96 well microtiter plate and dried at 45°C for 20 min. The dried wells were used as reaction vessels for the coupled "enzymatic" assay which employed α-galactosidase and galactose dehydrogenase as described previously [Schiweck and Busching, (1969) *Zucker* 22:377-384; Schiweck and Busching, (1975) *Zucker* 28:242-243; Raffinose Detection Kit, Boehringer Mannheim GMBH, Catalog Number 428 167] with modifications of the assay conditions. The modifications of the assay included addition of Bovine Serum Albumin (15 mg/mL) to the assay and α-galactosidase buffers, increasing the temperature and time of the α-galactosidase incubation from room temperature to 45°C and 30 min, and increasing the time of the galactose dehydrogenase incubation from 20 min to 60 min, and using stachyose instead of raffinose for the α-galactoside standard. After incubation, the absorbance at 340 nm of the samples was determined on a BIO-TEK® Model EL340 Microplate reader. The amount of α-galactosides present in the samples was determined by comparison to known quantities of the stachyose standard. To facilitate the analysis of thousands of samples, enzymatic assays were replicated once. Lines that appeared to be low in raffinose saccharide content from the primary assay were subsequently reassayed in triplicate, beginning from the ground seed, if sufficient material was available. Lines whose composition was confirmed in the secondary assay were grown to maturity under field conditions and seed from the field-grown plants were assayed again. In cases where more specific information about the raffinose saccharide and galactinol profile was required, low raffinose saccharide lines identified by the enzymatic assay were reassayed using the HPLC assay (described below).

To facilitate the rapid selection of low raffinose saccharide germplasm, a thin layer chromatography "TLC" assay was developed. For this assay, about 60 mg of ground seed powder was placed into a 13 x 100 mm screw top test tube, to which 1 mL of 4:3 (v/v) methanol:water and 1 mL of chloroform were added. The tubes were capped, placed in racks and mixed on a rotary shaker for 60 min at 25 rpm at room temperature. After extraction the tubes were centrifuged at 2100 rpm for 5 min. A 4 µL sample was taken from the methanol:water layer and placed on a 20 x 20 cm 'Baker' Silica Gel preadsorbent/channeled TLC plate with a 250 mm analytical layer. Samples were allowed to dry at room temperature and the plate was then placed in a TLC tank with a 3:4:4 (v/v/v) solution of Ethyl Acetate:Isopropanol:20% Acetic Acid (in water). The solution was allowed to soak up the analytical channels for 10 cm, at which time the plate was removed and allowed to air dry in a fume hood. The plate was then sprayed with an aniline-diphenylamine reagent to identify the carbohydrates in the sample. This reagent was prepared by mixing I mL of aniline with 100 mL of acetone, 1 gram of diphenylamine, and 10 mL of phosphoric acid. The plate was then placed in a 100°C oven for 15 min to dry and removed and allowed to cool down before reading the analytical channels. Stachyose and raffinose content in the soybean samples were estimated by comparison to the elution pattern seen in pure standards.

A high performance anion exchange chromatography/pulsed amperometric assay, referred to herein as the "HPLC" assay, was used for determining the content of individual raffinose saccharides (e.g., stachyose and raffinose), galactinol, and for confirming the results of either the enzymatic or TLC assays. Conditions for the grinding and extraction of the seed were identical to those used for the previous "enzymatic" assay. A 750 µL aliquot of the aqueous phase was removed and dried under reduced pressure at 80°C. The dried material was then dissolved in 2 mL of water and mixed vigorously for 30 sec. A 100 µL aliquot was removed and diluted to 1 mL with water. The sample was mixed thoroughly again and then centrifuged for 3 min at 10,000 x g. Following centrifugation, a 20 µL sample was analyzed on a Dionex™ PA1 column using 150 mM NaOH at 1.3 mL/min at room temperature. The Dionex™ PAD detector was used with E1 = 0.05 v, E2 = 0.60 v and E3 = -0.60 v and an output range of 3 mA. Galactinol, glucose, fructose, sucrose, raffinose, stachyose and verbascose were well separated by the chromatographic conditions. The carbohydrate content of the samples was determined by comparison to authentic standards.

Results obtained from the carbohydrate analyses were subjected to analysis of variance using the software SuperANOVA (Abacus Concepts, Inc., 1984 Bonita Avenue, Berkeley, CA 94704). When appropriate, Fisher's Protected LSD was used as the post-hoc test for comparison of means. In other comparisons, means were considered statistically significant if the ranges defined by their standard errors (SEM's) did not overlap. In cases where raffinose saccharide means were being compared to the mean of a control line, a mean was considered significantly lower than that of the control if said mean was at least three standard deviations below that of the control mean.

### Mutagenesis and Selection of Mutants

Approximately 130,000 seeds (22.7 kg) of LR13 (a line essentially identical to Williams 82) were soaked in 150 L of tap water under continuous aeration for eight hours. Aeration was accomplished by pumping air through standard aquarium "airstones" placed in the bottom of the soaking vessel. Imbibed seeds were drained and transferred to 98 L of a 2.5 mM N-nitroso-N-methylurea (NMU) solution buffered at pH 5.5 with 0.1 M phosphate buffer under continuous aeration. Seeds remained in the NMU solution for three hours and were then put through a series of rinses to leach out the remaining NMU. For the first rinse, treated seeds were transferred to 45 L of tap water for 1 min. For the second rinse, seeds were transferred to 45 L of fresh tap water under continuous aeration for one hour. For the third rinse, seeds were transferred to 45 L of fresh tap water under continuous aeration for two hours. For the fourth rinse, seeds were transferred to 45 L of fresh tap water under continuous aeration. One half of the seeds were removed from the fourth rinse after two hours (sub-population 1) while the other half of the seeds were removed from the fourth rinse after five hours (sub-population 2). After removal from the fourth rinse, seeds were drained of exogenous water and spread out on cardboard sheets to dry off in the sun for one hour. The imbibed M1 seeds were then field planted (Isabela, Puerto Rico, USA) in rows spaced 46 cm apart at a density of approximately 14 seeds per foot within the rows and a depth of 2.5 cm.

Two pools of M2 seeds (from sub-populations 1 and 2) were harvested in bulk from the M 1 plants. Approximately 40,000 M2 seeds from sub-population 1 and 52,000 M2 seeds from sub-population 2 were planted at Isabela, Puerto Rico, USA. Within each sub-population, five pods from each of 3,000 M2 plants were harvested and bulked to obtain a bulk M3 seed population. M3 bulks were planted at Isabela, Puerto Rico. At maturity, seed from 5000 M3 plants were harvested individually to obtain 5000 M3:4 lines from each sub-population.

During the winter of 1991, a total of at least 8,000 M3:4 lines were screened to measure the content of raffinose saccharides using the enzymatic method described above. In the intital screening, two lines with decreased total raffinose saccharides were identified which proved heritable in a second, selfed generation. One of these lines, designated LR33, had reduced levels of both stachyose and raffinose in comparison to elite soybean cultivars grown as controls in the same environment. In comparison to the average values for three elite cultivars grown in the same environment, the stachyose content of LR33 was statistically significantly lower. The soluble carbohydrate content of bulked seeds harvested from selfed lines derived from LR33 for three generations is shown in Table 1.

**TABLE I**

| Soluble carbohydrates in mature seeds of soybean lines derived by selfing line LR33 | | | | |
|---|---|---|---|---|
| LINE | STACHYOSE | RAFFINOSE | GALACTINOL | SUCROSE |
| LR33 | 61 | 18 | 0 | N.D.¹ |
| IST-83 | 38 | 11 | 0 | 153 |
| 1991 Elite (avg.) | 93 | 19 | 0 | N.D.¹ |
| 2ST-88 | 51 | 13 | 0 | 209 |
| 1992 Elite (avg.) | 68 | 12 | 0 | N.D.¹ |
| 3ST-101 | 25 | 8 | 0 | 126 |
| 1993 Elite (avg.) | 76 | 17 | 0 | N.D.¹ |
| (All values are in µmoles g⁻¹) | | | | |

| | | | | |
|---|---|---|---|---|
| ¹N.D. Not determined | | | | |

The subsequent lines (1ST-83, 2ST-88 and 3ST-101) were all derived by self pollination of LR33. Each of these lines had lower stachyose contents than did control lines.

### EXAMPLE 2

### IDENTIFICATION OF THE PHYSIOLOGICAL DEFECT RESPONSIBLE FOR THE LOW RAFFINOSE SACCHARIDE PHENOTYPE OF LR33

### Genetic crosses using LR33 as a low raffinose saccharide parent

In an effort to further reduce the total raffinose saccharide content of soybean seeds, genetic crosses were made between LR33 and LR28. Line LR28 is described in World Patent Publication WO93/07742. Briefly, it is also a low combined raffinose plus stachyose line discovered in a screening proceedure very similar to that described in Example 1. The low raffinose and stachyose content is consistent from one generation to the next. In addition, the galactinol content of the mature seeds of line LR28 and lines derived from it by crossing to other parents, is greatly elevated relative to wild type soybean seeds.

F 1 plants from the cross of LR33 and LR28 were grown and self pollinated to produce segregating F2 progeny. Seeds of LR28-derived lines, elite cultivars and the segregating population resulting from the cross of LR28 and LR33 were planted in the same environment. F2:3 seeds were harvested from each F2 plant and screened for α-galactoside content using the enzymatic assay described above. Low α-galactoside selections from the preliminary screen were advanced to the HPLC assay to obtain complete raffinose saccharide profiles. Carbohydrate profiles from typical lines selected for low total α-galactoside content are shown in Table 2.

**TABLE 2**

| Soluble carbohydrates in mature seeds of soybean lines derived from crosses of line LR28 and LR33 and subsequent backcrosses to elite cultivars (LR33 and 5ST-1003 (an LR28 derived line) are included as examples of the cross parents; 2242 as an elite cultivar control.) | | | | |
|---|---|---|---|---|
| LINE | STACHYOSE | RAFFINOSE | GALACTINOL | SUCROSE |
| LR33 | 61 | 18 | 0 | N.D.¹ |
| 5ST-1441 | 57 | 18 | 0 | N.D.¹ |
| 5ST-1434 | 6 | 15 | 0 | 216 |
| 5ST-1309 | 0 | 5 | 0 | 287 |
| 2242 | 75 | 18 | 0 | 168 |
| 5ST-1003 | 19 | 4 | 65 | 200 |
| (All values are in µmoles g⁻¹) | | | | |

| | | | | |
|---|---|---|---|---|
| ¹ N.D. Not determined | | | | |

5ST-1441, 5ST-1434, and 5ST-1309 were all derived from the LR33 by LR28 cross. While line 5ST-1441 closely resembles the LR33 parent, surprisingly lines 5ST-1434 and 5ST-1309 are much lower in both raffinose and stachyose than either parental line and do not contain the elevated galactinol level characteristic of line LR28.

### In vitro assay of activity of enzymes in the raffinose saccharide pathway

In studies designed to find the cause of the unexpected raffinose, stachyose and galactinol phenotype observed in some lines derived from the LR33 by LR28 cross, several enzyme activities and metabolite pools were measured in soybean seeds harvested just before the physiologically mature stage during the period of rapid raffinose saccharide biosynthesis. Seeds were removed from pods that had just begun to yellow. Seeds from such pods that were also loosing green color or just beginning to yellow themselves were chosen for assay of the last three enzymes in raffinose saccharide biosynthesis (See Figure 1).

Seeds were removed from the pod, weighed to obtain fresh weight, then ground in a mortar and pestle in ten volumes of 50 mM HEPES-NaOH, pH 7 buffer which was also 5 mM in 2-mercaptoethanol. The ground samples were centrifuged at 10,000 x g for 10 min and the supernatant was desalted by passage through Sephadex G-25 which had been equilbrated in the grinding buffer.

For the assay of galactinol synthase, 10 µL of the desalted extract was added to 90 µL of the pH 7 HEPES buffer which was also 20 mM in *myo*-inositol, 10 mM in dithiothreitol, 1 mM in MnCl₂ and 1 mM in UDP-[¹⁴C]galactose (1.25 µCi µmol⁻¹). The assay mixture was incubated for 10 min at 25°C then stopped by the addition of 400 µL of ethanol. To the stopped reactions was added 200 µL of Dowex AG-1x8 anion exchange resin (BioRAD) and the mixture was shaken for 25 min. The resin was removed by centrifugation and the supernatant was taken for scintillation counting. Non-anionic radioactivity was taken as a measure of galactinol synthesis.

For the assay of raffinose synthase and stachyose synthase, 50 µL of the desalted extract was added to 50 µL of 25 mM HEPES buffer at pH 7 that was also 10 mM in dithiothreitol, 10 mM in galactinol, and 40 mM in sucrose for the assay of raffinose synthase or 40 mM in raffinose for the assay of stachyose synthase. After a 1 h incubation at 25°C, the reactions were stopped by the addition of 40 µL of ethanol and then placed in a boiling water bath for 1 min to precipitate proteins. The reaction mixes were centrifuged to clear, the supernatant was passed through a 0.22 micron filter and then reduced to dryness under vacuum. The residue was re-dissolved in 0.5 mL of water and 20 µL were separated on the Dionex HPLC system as described above for the quantitation of raffinose and stachyose. Results of an experiment done using seeds harvested from field grown plants in August of 1994 are given in Table 3.

**TABLE 3**

| Activities of galactinol synthase, raffinose synthase, and stachyose synthase in yellowing seeds of three soybean lines (The wildtype control is line 1923. Enzyme activities are expressed as µmole of product produced per gram of fresh seed weight per hour under the assay conditions. Values are mean ± standard deviation for five replicates.) | | | |
|---|---|---|---|
| LINE | GALACTINOL SYNTHASE | RAFFINOSE SYNTHASE | STACHYOSE SYNTHASE |
| 1923 | 7.5±4.1 | 0.10±0.05 | 0.65±0.18 |
| LR28 | 16.5±5.2 | 0.004±0.007 | 0.81±0.24 |
| LR28xLR33 | 22.6±8.8 | 0.006±0.008 | 0.87±0.21 |

Of the three enzymes committed to raffinose and stachyose synthesis, only raffinose synthase shows a clear decrease in activity in the mutant, low raffinose saccharide lines in comparison to wild type. A mutation causing decreased activity of raffinose synthase is consistent with the position of that enzyme in the biosynthetic pathway and the accumulation of galactinol observed in lines which carry only LR28 as the source of the low raffinose saccharide phenotype. Galactinol and sucrose are the two substrates for raffinose synthase and both metabolites accumulate in LR28 containing lines (see Figure 1 and Table 2).

Despite the lower levels of stachyose, raffinose and galactinol obtained when LR33 lines were crossed with LR28 lines, the reduced raffinose synthase activity conferred by the LR28 mutation is the only altered activity. While decreased galactinol synthase activity seemed a likely candidate for the lesion in the LR33 line due the decreased galactinol content conferred by the mutation when in combination with LR28, there is no decrease in the measured, *in vitro* activity of that enzyme.

### Assay of the free myo-inositol content of maturing soybean seeds

While there was no decrease in galactinol synthase *in vitro* in the LR33 by LR28 cross, the possibility that the *in vivo* activity of galactinol synthase in maturing seeds of LR33 derived lines is limited by the availability of one of its substrates was checked. The supply of UDP-galactose to galactinol synthase could be decreased by mutations in UDP-glucose 4' epimerase and the supply of *myo*-inositol could be limited by mutation effecting either its synthase or the specific phosphatase that produces the free inositol form (Figure 1). The *myo*-inositol pool was checked by assaying for the total free *myo*-inositol content in wild type and LR33 derived seeds. Three soybean lines, a wild type cultivar A2872, and two LR33xLR28-derived lines, 5ST-1434 and 5ST-1309, were grown in a growth room under 16 h days with a day/night temperature regime of 30°C/22°C. Seeds were taken from pods that had just begun to yellow and that were themselves turning from very light green to yellow. Three seeds from each line were ground in 8 mL of 80% methanol. The mixture was centrifuged at 12,000 x g for 15 min and the supernatant decanted to a 50 mL flask. The extraction was repeated twice and the supernatants combined. The combined supernatants were reduced to dryness under vacuum at 40°C and the residues re-dissolved in I mL of water. The re-dissolved extracts were deionized by passage through 3 mL of mixed bed ion exchange resin (BioRad AG501x8) and the through flow plus 4 mL of wash was again reduced to dryness. The residue was re-dissolved in 500 µL of water and a 70 µL aliquot was separated by HPLC on a Zorbax Amino column (DuPont) eluted at 1 mL min⁻¹ with 70% acetonitrile. Sugars in the column eluate were detected by differential refractive index. Separation of standard mixtures of sucrose and *myo*-inositol showed that inositol emerges later and only partially separated from sucrose. For analysis of the seed extracts, 1 mL of eluate following the sucrose peak was trapped for re-injection on the Dionex PAD system described in Example 1. In the Dionex system, *myo*-inositol emerges well before sucrose and could be cleanly separated from sucrose contaminating the Zorbax Amino column fraction. By comparison to external standards of *myo*-inositol, the 5 µL injection from the wild type 2872 line contained 3.2 nmoles of *myo*-inositol while 5 µL injections from 5ST-1434 and 5ST-1309 contained 0.39 and 0.23 nmoles respectively.

A second experiment was performed to further characterize the difference in free *myo*-inositol content of wild type and LR33 derived seeds. Seven single seeds from a second control line (2242) and from the LR33 derived line 5ST-1434 were harvested according to the maturity criteria described above. The seeds were individually weighed, placed in 1.5 mL microfuge tubes, crushed with a spatula, frozen on dry ice and lyophilized. The dry residue was weighed and 1 mL of 80% methanol which contained 1 µmole of trehalose was added to act as a *myo*-inositol retention marker on the Zorbax amino column and as an internal standard. The extraction media was heated for 1 h at 60°C, re-ground with a small pestle in the microfuge tube and centrifuged to pellet the insoluble material. The supernatant was transferred to a second microfuge tube containing about 100 µL of mixed bed resin, the mixture was briefly shaken and 20 µL of the solution above the resin was removed and taken to dryness under vacuum. The residue was re-dissolved in 110 µL total volume and 55 µL were injected onto the Zorbax Amino column run as described above. The trehalose peak was trapped and 20 µL of the column fraction was reinjected on the Dionex system. The *myo*-inositol peak was quantitated by comparison to the trehalose internal standard. The mean ± standard deviation for the wild type *myo*-inositol content was 2.18±0.98 µmole (g dry wt)⁻¹ while the 5ST-1434 seeds contained 0.77±0.32 µmole (g dry wt)⁻¹. Both experiments indicate that seeds which carry the LR33 mutation contain less *myo*-inositol during the period of rapid raffinose saccharide synthesis. In neither experiment was the total *myo*-inositol pool totally absent. Such experiments can only measure the total seed pool of metabolites however and *myo*-inositol has other fates which could further limit its use by galactinol synthase (Figure 1).

### The effect of myo-inositol perfusion on the in vivo labeling of raffinose saccharides

To ascertain whether or not the observed decrease in free *myo*-inositol content of the LR33 derived seeds might be the cause of the decreased raffinose saccharide content at maturity, tissue slice feeding studies were performed. Four seeds each from wild type line 2242 and LR33-derived line 5ST-1434 were harvested by the maturity criterion described above. The seed coats were removed and the cotyledons rinsed in 5 mM potassium phosphate buffer at pH 5.5 then sliced into approximately I mm slices and again rinsed with buffer. The tissue slices were divided into two groups. One group was immersed in potassium phosphate buffer and the second group was immersed in the potassium phosphate buffer containing 50 mM *myo*-inositol. The tissue slices were vacuum-infiltrated and incubated for 30 min at room temperature. After the pre-incubation period, 5 µCi of ¹⁴C-glucose was added to each grouping and the tissue was again vacuum-infiltrated. Ten tissue slices from each group were taken at 2 h and at 8 h after addition of the labeled glucose. The tissue slices were placed in tarred 1.5 mL microfuge tube to obtain fresh weight and ground in 300 µL of 80% methanol. The tubes were centrifuged, the supernatant removed to a second tube, the extraction was repeated twice more and the supernatants were combined. The combined supernatants were reduced to dryness under vacuum, re-dissolved in 50% acetonitrile and separated on the Zorbax amino HPLC system. Fractions were collected for scintillation counting at 15 second intervals through the region of the chromatogram containing glucose, sucrose, raffinose and galactinol and at 1 min intervals through the region containing stachyose. Radioactive fractions corresponding to the sugar standard peaks were grouped for analysis. The results are expressed as per cent of the radiolabel in the four product sugars in Table 4.

**Table 4**

| Radiolabel in sucrose, galactinol, raffinose and stachyose expressed as per cent of label in those sugars combined for control and an LR33 derived line with and without pre-incubation with *myo*-inositol | | | | | | | | |
|---|---|---|---|---|---|---|---|---|
| WILD TYPE TISSUE SLICES | | | | | 5ST-1434 TISSUE SLICES | | | |
| | 2 h | 2h +inos | 8 h | 8 h +inos | 2 h | 2 h +inos | 8 h | 8h +inos |
| SUCROSE | 52.4 | 33.4 | 41.0 | 23.8 | 86.8 | 43.5 | 79.8 | 21.8 |
| GALACTINOL | 13.4 | 24.8 | 8.0 | 11.1 | 3.2 | 27.6 | 1.5 | 9.5 |
| RAFFINOSE | 19.8 | 20.7 | 17.3 | 17.7 | 4.5 | 15.2 | 3.1 | 13.0 |
| STACHYOSE | 14.4 | 21.0 | 33.6 | 47.4 | 5.4 | 13.7 | 15.9 | 55.6 |

Both wild type and 5ST-1434 tissue slices convert label from supplied ¹⁴C-glucose to sucrose and then into galactinol, raffinose, and stachyose. Without the addition of exogenous *myo*-inositol, the line containing the LR33 mutation (5ST-1434) converts very little label into the raffinose saccharides (20.5% after 8 h) in comparison to the wild type line (58.9% after 8 h). Both genotypes respond to exogenous *myo*-inositol by converting more of the supplied label to raffinose saccharide sugars. With the addition of *myo*-inositol the two genotypes become essentially equal in their ability to convert the supplied label first into galactinol and then into raffinose and stachyose. The rate of conversion is increased even in the wild type line in comparison to the non-infused control.

It appears that the supply of *myo*-inositol to galactinol synthase is always a limitation to the rate at which raffinose and stachyose can be synthesized. The presence of the LR33 mutation makes that limitation much greater.

The labeling pattern is surprising in that there is no buildup of label in galactinol as would be expected if the LR28 mutation in raffinose synthase were still effective in slowing flux through that step in the combined mutant lines. While the addition of *myo*-inositol can be expected to increase flux through galactinol synthase to galactinol, the label should have accumulated there due to the decreased activity in raffinose synthase. The absence of that accumulation calls into question either the effectiveness or the presence of the LR28 mutation in this line.

### The influence of the LR33 mutation on seed phytic acid and inorganic phosphate levels

The above results suggest that the LR33 mutation resides before free *myo*-inositol in the pathway shown in Figure 1. To further limit the possible site of the mutation, other metabolites in that portion of the pathway were measured.

Since soybean seeds contain phytic acid at levels of 20 to 30 µmoles g⁻¹ at maturity and since 50 to 70% of the total seed phosphate is contained in phytic acid [Raboy et al. (1984) *Crop Science 24*:431-434], it seemed likely that mutations effecting *myo*-inositol synthesis might also effect seed phytic acid and inorganic phosphate levels. The levels of phytic acid and inorganic phosphate in three wild type soybean lines and three lines that contain the LR33 mutation were assayed in dry seeds by a modification of the method described by Raboy [*supra*]. Approximately twenty seeds from each line were ground in a small impact mill and 100 mg of the resulting powder was weighed into a 15 mL screw cap tube. Five mL of 0.4 N HCl with 0.7M Na₂SO₄ was added to the powder and the mixture was shaken overnight on a rocker platform. The tubes were centrifuged at 3900 x g for 15 min and 2 mL of the supernatant was removed to second, glass tube. Two mL of water and 1 mL of 15 mM FeCl₂ were added and the tubes were heated for 20 min in a boiling water bath. The tubes were centrifuged as above to precipitate the iron:phytic acid complex and the supernatant was discarded. The pellets were resuspended in 2 mL of 0.2 N HCl and heated for 10 min in the boiling water bath. The precipitate was again removed by centrifugation and re-suspended in 2 mL of 5 mM EDTA.

Fifteen µL of the EDTA suspension was taken for wet ashing to obtain phytic acid phosphate. To each tube containing the 15 µL aliquot was added 10 µL of a 10% solution of calcium nitrate. The water was allowed to evaporate and the residue was heated in a flame until a white ash remained in the tube. The ash was dissolved in 0.3 mL of 0.5 N HCL and heated at 90°C for 20 to 30 min. Acid molybdate reagent (0.7 mL of 0.36% ammonium molybdate and 1.42% ascorbic acid in 0.86 N sulfuric acid) was added and the color was allowed to develop for 1 h before reading at 820 nm.

Inorganic phosphate was determined by adding 0.3 mL of 0.5 N HCl and 0.7 mL of the phosphate color reagent to either 20 or 40 µL aliquots of the initial 5 mL extract. Potassium phosphate standards were developed at the same time. The experiment was repeated three times and the results are given in Table 5.

**Table 5**

| Seed phosphate in inorganic phosphate and in phytic acid expressed as µmoles phosphate g⁻¹ (Values are mean ± standard deviation where more that two replicates were obtained or the average value when two replicates were obtained.) | | | | |
|---|---|---|---|---|
| SEED LINE | GENOTYPE | REPLICATES | INORGANIC PHOSPHATE | PHYTIC ACID PHOSPHATE |
| 2872 | WILD TYPE | 7 | 2.7±3 | 125±8.4 |
| 1923 | WILD TYPE | 1 | 2.0 | 126 |
| 1929 | WILD TYPE | 2 | 1.5 | 155 |
| 5ST-1309 | LR33 | 4 | 76.0±5.7 | 62.5±12.4 |
| GxE-117 | LR33 | 7 | 36.7±6.4 | 55.8±13.9 |
| GxE-76 | LR33 | 6 | 32.2±3.5 | 72.8±14 |

The LR33 mutation also causes a decrease in seed phytic acid level (expressed as phosphate in the phytic acid fraction) of about two fold with a concomitant increase in the seed inorganic phosphate content of about 15 to 25 fold depending upon the genetic background in which the mutation is contained. Based on the pathway in Figure 1, the decreased phytic acid content, along with the decreased free *myo*-inositol content are most likely to be caused by a mutation decreasing the activity of *myo*-inositol 1-phosphate synthase.

The exact cause of the increase in the inorganic phosphate content is not known, however it has long been assumed that phytic acid functions as a phosphate storage form in seeds and other parts of the plant. It is possible that when the preferred storage platform (*myo*-inositol) is unavailable, incoming inorganic phosphate has no other major fate and simply accumulates.

### EXAMPLE 3

### IDENTIFICATION OF THE SEED PHENOTYPE OF THE HOMOZYGOUS LR33 LINE

Several additional soybean lines were analyzed for seed inorganic phosphate. One hundred mg of ground seed was extracted with 1 mL of hot water and centrifuged to remove the insoluble matter. The supernatant was extracted with 100 µL of methylene chloride to remove lipid material and the remainder of the aqueous extract was made to 10% with trichloroacetic acid. The solution was centrifuged to remove proteins and 5 µL of the supernatant was analyzed for inorganic phosphate as described in Example 2. Table 6 gives the results of those assays.

**Table 6**

| The inorganic phosphate content (µmole g⁻¹) of seeds from wild type, LR28, LR33 and LR28xLR33 soybean plants | | |
|---|---|---|
| LINE | GENOTYPE | INORGANIC PHOSPHATE |
| A2872 | wildtype | 12.7 |
| LR28 | LR28 | 12.7 |
| LOW2 | LR28 | 12.9 |
| 5ST-1190 | LR28 | 14.4 |
| 5ST-1191 | LR28 | 14.0 |
| 5ST-1441 | LR33 | 23.4 |
| 5ST-1309 | LR28xLR33 | 117.6 |
| 5ST-1310 | LR28xLR33 | 113.9 |
| 5ST-1433 | LR28xLR33 | 74.2 |
| 5ST-1434 | LR28xLR33 | 57.1 |
| GxE-117 | LR28xLR33 | 74.3 |
| GxE-305 | LR28xLR33 | 70.7 |
| GxE-76 | LR28xLR33 | 79.1 |
| GxE-77 | LR28xLR33 | 58.4 |

All of the lines which contain the LR28 mutation alone have inorganic phosphate levels equal to the wild type controls. Line 5ST-1441 was derived from LR33 (Example 2 above) and has the moderate reduction in stachyose that was originally associated with the mutation. 5ST-1441 has only a slightly increased inorganic phosphate content. Despite the fact that neither parent is high in inorganic phosphate, all the lines derived form the cross of LR33 by LR28 have greatly elevated levels of inorganic phosphate.

The intermediate inorganic phosphate phenotype of line 5ST-1441 that contains only the LR33 mutation was unexpected. It may indicate that both the LR28 and the LR33 mutation must be present to produce the low phytic acid/high inorganic phosphate phenotype. If however the two mutations are in fact in the structural genes encoding the two activities that seem to be decreased, they are not related in the metabolic pathway in a manner that would suggest an additive effect on alteration of phytic acid synthesis. If the mutation in LR33 effects either free or total *myo*-inositol production, it alone should be responsible for the low phytic acid and resultant high inorganic phosphate phenotype.

An alternate explanation may be that the line 5ST-1441 is not homozygous for the LR33 mutation and that analysis of the bulk seed gives only the average phenotype for the wild type, homozygous mutant and heterozygous seeds. To check this possibility, thirteen single seeds from the bulk sample of 5ST-1441 seed were weighed, ground and extracted with hot water. Protein was not precipitated and inorganic phosphate was measured as before. The results are shown in Table 7.

**Table 7**

| Approximate inorganic phosphate content (µmoles g⁻¹) for thirteen individual seeds form line 5ST-1441 (Actual values are elevated in comparison to the results of Table 6 due to the presence of protein in the samples.) | |
|---|---|
| SEED NUMBER | INORGANIC PHOSPHATE |
| 5ST-1441-a | 21.9 |
| 5ST-1441-b | 30.4 |
| 5ST-1441-c | 25.4 |
| 5ST-1441-d | 81.1 |
| 5ST-1441-e | 29.7 |
| 5ST-1441-f | 25.7 |
| 5ST-1441-g | 25.5 |
| 5ST-1441-h | 30.8 |
| 5ST-1441-i | 28.2 |
| 5ST-1441-j | 123.5 |
| 5ST-1441-k | 27.9 |
| 5ST-1441-1 | 115.9 |
| 5ST-1441-m | 27.6 |

Seeds lettered d, j, and I have about three fold more inorganic phosphate than the remaining ten seeds analyzed. The ratio approximates that expected from a segregating population of seeds in which the mutant phenotype of high inorganic phosphate is recessive and due to the action of a single gene. To check this hypothesis, twenty seeds from the bulk sample of 5ST-1441 were imbibed in water at room temperature for 6 h. Excess water was blotted away and a sample of the cotyledons at the end away from the embryonic axis was cut off. The tissue pieces were weighed, placed in 1.5 mL microfuge tubes and ground in sufficient 70% methanol to give 10 mg fresh wt per 100 µL of extracted volume. Phosphate in the supernatant present after centrifugation was measured as described above and the results are shown in Table 8.

**Table 8**

| The inorganic phosphate content of 20 partial seeds from a segregating population of seeds from the line 5ST-1441 (Results are expressed as µmole per g fresh weight (µmole gfwt⁻¹).) | |
|---|---|
| SEED NO. | PO₄ (µmole gfwt⁻¹) |
| 1 | 4.9 |
| 2 | 4.3 |
| 3 | 5.3 |
| 4 | 40.1 |
| 5 | 6.1 |
| 6 | 5.0 |
| 7 | 37.1 |
| 8 | 9.9 |
| 9 | 5.9 |
| 10 | 3.8 |
| 11 | 26.9 |
| 12 | 4.7 |
| 13 | 5.5 |
| 14 | 3.9 |
| 15 | 5.2 |
| 16 | 17.7 |
| 17 | 5.9 |
| 18 | 4.9 |
| 19 | 2.5 |
| 20 | 28.9 |

Seeds 4, 7, 11, 16 and 20 were planted in pots in a growth room. Seeds 7, 11, 16, and 20 survived and were grown to maturity under the growth conditions described in Example 2. Bulk seeds from the mature plants and two wild type controls (A2872) were harvested after dry down and twenty seeds from each plant were ground in bulk for analysis of soluble carbohydrates and phytic acid using the methods described in Examples 1 and 2. The total seed phytic acid content was calculated as the (phytic acid phosphate content)/6. The results are shown in Table 9.

**Table 9**

| Soluble carbohydrate content and phytic acid content (expressed as µmole g⁻¹) for four plants from a segregating population of seeds carrying the LR33 mutation and selected for elevated inorganic phosphate in partial seed analysis along with two control plants (Phytic acid values are the average of two replicates.) | | | | | |
|---|---|---|---|---|---|
| LINE | PHYTIC ACID | STACHYOSE | RAFFINOSE | GALACTINOL | SUCROSE |
| A2872 | 32.3 | 71 | 19 | 3 | 166 |
| A2872 | 30.8 | 67 | 24 | 0 | 144 |
| LR33-7 | 23.7 | 40 | 16 | 0 | 155 |
| LR33-11 | 8.8 | 4 | 13 | 0 | 212 |
| LR33-16 | 9.5 | 4 | 13 | 2 | 209 |
| LR33-20 | 7.6 | 4 | 11 | 0 | 208 |

While the soluble carbohydrate phenotype of the plant grown from seed number 7 (LR33-7) is very similar that ascribed to the LR33 mutation as shown in Table 1, the other plants have much lower levels of the raffinose saccharides and have much higher levels of sucrose. These levels of soluble carbohydrate are very similar to the phenotype ascribed to some plants in the mutant combination LR28xLR33 in Table 2. The most probable explanation for this discrepancy is that the bulk seed analyzed to give the data in Table 1 came from plants segregating for the LR33 mutation rather than plants homozygous for the mutation. When plants homozygous for the mutation are selected, as was done in the case of plants 11, 16 and 20 in this example, the true, homozygous seed phenotype is observed. The previous assumption that both the LR28 and the LR33 mutations needed to be present to produce the combination of low raffinose, stachyose and galactinol was incorrect. While lines homozygous for the LR33 mutation were not obtained from selfing the originally identified mutant line, they were obtained from segregants arising from the cross of that line with LR28. While some of the progeny of the cross likely do contain both mutations, the phenotype from the LR33 mutation is dominant to that arising from the LR28 mutation. That dominance stems primarily from the location of the LR33 mutation upstream in the carbon flow from the LR28 mutation (see Figure 1 and Example 2).

The reason that the homozygous LR33 phenotype remained elusive may be due to germination problems encountered with the original mutant line. In bulk field segregation conditions the loss of 25% (that fraction that would have been homozygous for the mutation) of the planted seed from selfing LR33 could have been missed. Harvested plants from the bulk population would have then been depleted in homozygotes but the mutant gene would be retained in the population in the heterozygous state. We surmise that out crossing to a genetic background more conducive to allowing LR33 homozygotes to emerge in field conditions allowed recovery of the homozygote. That the initial out crosses were to lines carrying another mutation in the raffinose saccharide biosynthetic pathway were incidental to the original intent of the cross and not essential either for the superior phenotype or the improved field emergence.

The LR33 mutation is thus capable of producing soybean plants that produce seeds with less than 5 µmoles of stachyose per gram of seed, less than 20 µmoles of raffinose per gram of seed, more than 200 µmoles of sucrose per gram of seed and less than 10 µmoles of phytic acid phosphate per gram of seed.

### EXAMPLE 4

### SOLUBLE CARBOHYDRATE AND PHYTIC ACID CONTENT OF A SOYBEAN LINE CONTAINING THE LR33 MUTATION

The low raffinose saccharide lines LR33 and LR28 were crossed and segregating F2 plants were selected for low levels of raffinose, stachyose and galactinol in the seed of the F2 plants by the HPLC assay described in Example 1. Selected lines derived from this cross were then crossed to elite cultivar parents and the progeny of those crosses were selected in the F2 generation by the same process. Among these lines containing low levels of raffinose saccharides, several were chosen for advancement based on the agronomic characteristics of the vegetative plant. One such line, designated 4E76, was subsequently test crossed to another elite soybean cultivar and single seeds from the selfed F 1 plant derived from that cross were analyzed for soluble carbohydrates and for inorganic phosphate content. The seeds fell into two classes, those with very low inorganic phosphate and wild type levels of raffinose plus stachyose, and a smaller class of seeds with elevated levels of inorganic phosphate and very low levels of raffinose plus stachyose. The ratio of these two classes was very near the 3:1 ratio expected of the recessive LR33 mutation. The was no evidence of segregation of LR28 which is the other mutation effecting raffinose saccharides that was present in the original cross. Line 4E76 thus represents the LR33 mutation in a selected elite cultivar background. The line was grown in a total of nine environments over two years along with selected elite check lines for carbohydrate analysis. A more limited set of samples from three environments was analyzed for phytic acid content. The carbohydrate data is shown in Table 10 and the phytic acid data in Table 11.

**Table 10**

| Mean, standard deviation (SD) and two standard deviations (2SD) of the mean for the stachyose, raffinose and sucrose content of LR33-containing line 4E76 and thirteen elite cultivar checks (All values are expressed as µmoles g⁻¹ seed weight.) | | | | | | | | | |
|---|---|---|---|---|---|---|---|---|---|
| LINE | STACHYOSE | | | REFFINOSE | | | SUCROSE | | |
| | MEAN | SD | 2SD | MEAN | SD | 2SD | MEAN | SD | 2SD |
| 4E76 | 2.4 | 0.5 | 1.0 | 6.9 | 0.7 | 1.4 | 249 | 42.9 | 85.8 |
| A2514 | 44.8 | 2.7 | 5.4 | 13.0 | 2.6 | 5.2 | 138 | 9.6 | 19.2 |
| A2396 | 47.8 | 1.1 | 2.2 | 9.6 | 0.9 | 1.8 | 158 | 9.6 | 19.2 |
| A1923 | 50.2 | 2.8 | 5.6 | 8.6 | 0.5 | 1.0 | 148 | 9.4 | 18.8 |
| A2506 | 51.0 | 3.7 | 7.4 | 16.4 | 4.0 | 8.0 | 161 | 28.2 | 56.4 |
| A3322 | 54.2 | 7.3 | 14.6 | 14.0 | 2.7 | 5.4 | 147 | 27.1 | 54.2 |
| A2923 | 56.4 | 2.2 | 4.4 | 13.6 | 1.7 | 3.4 | 125 | 9.2 | 18.4 |
| A2234 | 57.2 | 9.1 | 18.2 | 16.6 | 3.4 | 6.8 | 145 | 21.2 | 42.4 |
| A1923 | 57.5 | 4.5 | 9.0 | 18.5 | 3.4 | 6.8 | 156 | 29.8 | 59.6 |
| A3313 | 61.0 | 9.7 | 19.4 | 14.4 | 3.9 | 7.8 | 172 | 37.9 | 75.8 |
| A1662 | 61.6 | 6.2 | 12.4 | 13.8 | 3.4 | 6.8 | 127 | 21.4 | 42.8 |
| A3935 | 62.8 | 9.2 | 18.4 | 11.6 | 2.1 | 4.2 | 184 | 38.7 | 77.4 |
| A2833 | 63.8 | 8.6 | 17.2 | 15.4 | 2.4 | 4.8 | 150 | 38.7 | 77.4 |
| A3510 | 68.8 | 9.4 | 18.8 | 14.2 | 1.9 | 3.8 | 160 | 33.0 | 66.0 |

**Table 11**

| Mean, standard deviation and two standard deviations of the mean for the phytic acid content of the LR33-containing line 4E76 and the elite cultivar check A2872. (All values are expressed as µmoles g⁻¹ seed weight and represent data from three environments.) | | | |
|---|---|---|---|
| LINE | PHYTIC ACID | | |
| | MEAN | SD | 2SD |
| 4E76 | 12.3 | 2.4 | 4.8 |
| A2872 | 20.9 | 0.9 | 1.8 |

In comparison to elite soybean cultivars that are typical of commercial soybeans, the LR33-derived line is eight to nine fold lower in the mass of total raffinose saccharide per gram of seed weight. The mass of phytic acid is also decreased by about 40%.

In comparison to elite soybean cultivars, the LR33-derived lines are lower in the mass of both raffinose and stachyose. While raffinose content is only slightly lower than values seen in elite lines, stachyose content is very greatly reduced. It is known that the detrimental effect of soybean raffinose saccharide content on energy use efficiency when soybean meal is fed to monogastric animals is due to the poor digestibility of the α-galactosidic bond. Accordingly, a decrease in combined raffinose plus stachyose content is an appropriate measure of increased digestibility. In fact, this measurement may even underestimate the effect of the instant phenotype since stachyose contains two moles of the α-galactosidic bond per mole of sugar.

The absolute raffinose saccharide content of mature soybeans is known to vary in response to environmental factors. The effect of the mutation present in LR33-derived lines is of sufficient magnitude to render the combined raffinose plus stachyose content of such lines below 14.5 µmol/g seed weight and should always remain far below that of wild type soybeans grown in the same environment.

The phytic acid content of mature soybean seeds is also known to vary in response to environmental factors, primarily due to variation of levels of available phosphate in soil. Once again, the mutation present in LR33-derived lines maintains total seed phytic acid content below 17 µmol/g across all growth environments.

### EXAMPLE 5

### MOLECULAR IDENTIFICATION OF THE LR33 MUTATION

The evidence from analysis of metabolites in the LR33 derived lines presented in Example 2 strongly suggests that the LR33 mutation causes a decrease in the ability of the seed to produce *myo*-inositol.

In plants as well as other organisms, *myo*-inositol is produced solely by a pathway which begins with the conversion of glucose-6-phosphate to *myo*-inositol 1-phosphate in a reaction catalyzed by *myo*-inositol 1-phosphate synthase and ends with the hydrolysis of the 1-phosphate by a specific *myo*-inositol-phosphate phosphatase (see Figure 1; Loews, F.A. In: *Inositol Metabolism in Plants* (1990) Wiley-Liss, New York, pp 13-19]). Since the phytic acid has as its probable precursor *myo*-inositol 1-phosphate and since the level of phytic acid is decreased by the LR33 mutation, the gene and gene product for *myo*-inositol-phosphate synthase was characterized in both wild type and LR33 mutant plants.

### cDNA cloning of the wild type soybean myo-inositol 1-phosphate synthase

A cDNA library was made as follows. Soybean embryos (ca. 50 mg fresh weight each) were removed from their pods and frozen in liquid nitrogen. The frozen embryos were ground to a fine powder in the presence of liquid nitrogen and then extracted by Polytron homogenization and fractionated to enrich for total RNA by the method of Chirgwin et al. ((1979) *Biochemistry 18*:5294-5299).

The nucleic acid fraction was enriched for poly A⁺ RNA by passing total RNA through an oligo-dT cellulose column and eluting the poly A⁺ RNA with salt as described by Goodman et al. ((1979) *Meth. Enzymol. 68*:75-90). cDNA was synthesized from the purified poly A⁺ RNA using cDNA Synthesis System (Bethesda Research Laboratory, Gaithersburg, MD) and the manufacturer's instructions. The resultant double-stranded DNA was methylated by Eco RI DNA methylase (Promega) prior to filling in its ends with T4 DNA polymerase (Bethesda Research Laboratory) and blunt-end ligation to phosphorylated Eco RI linkers using T4 DNA ligase (Pharmacia). The double-stranded DNA was digested with Eco RI enzyme, separated from excess linkers by passage through a gel filtration column (Sepharose CL-4B), and ligated to lambda ZAP vector (Stratagene) according to manufacturer's instructions. Ligated DNA was packaged into phage using the Gigapack™ packaging extract (Stratagene) according to manufacturer's instructions. The resultant cDNA library was amplified as per Stratagene's instructions and stored at -80°C.

Following the instructions in the Lambda ZAP Cloning Kit Manual (Stratagene), the cDNA phage library was used to infect *E. coli* XL1 cells and a total of approximately 300,000 plaque forming units were plated onto six 150 mm diameter petri plates.

Duplicate lifts of the plates were made onto nitrocellulose filters (Schleicher & Schuell, Keene, NH). The filters were prehybridized in 25 mL of hybridization buffer consisting of 6X SSPE, 5X Denhardt's solution, 0.5% SDS, 5% dextran sulfate and 0.1 mg/mL denatured salmon sperm DNA (Sigma Chemical Co.) at 60°C for 2 h.

The blocked filters were then hybridized to a radiolabelled probe made from a cDNA from *Arabidopsis thaliana* which had been identified as a *myo*-inositol-1-phosphate synthase by homology to yeast *myo*-inositol-1-phosphate synthase [Johnson, M.A. (1994) *Plant Physiol. 105*:1023-1024]. The *Arabidopsis* clone was obtained from the Arabidopsis Biological Resource Center, DNA Stock Center, 1060 Carmack Road, Columbus, OH 43210-1002, clone number 181C18T7113E9T7. The 1.2 kB cDNA insert was removed from the vector DNA by digestion with Sal I and Not I followed by agarose gel purification of the DNA fragment. The purified fragment was labeled with [³²P]dCTP with a random primer labeling kit (Bethesda Research Laboratory). The filters were allowed to hybridize overnight under the same conditions as described for pre-hybridization. Excess radiolabel was washed from the filters in 0.6x SSC containing 0.1% SDS. Two washes of about 10 min each in 0.2xSSC, 0.1% SDS at 60°C were then applied to remove non-specifically bound label and the filters were used to expose photographic film in an overnight exposure. Approximately 200 positive signals were observed. Six were purified by excision of the area around the signal, re-plating the phage and re-screening as above. Two clones were excised to phagmids and used to infect *E. coli* to obtain plasmid clones using the protocols described by the manufacturer (Strategene). Of the two clones, one designated p5bmi-1-ps was sequenced using Applied Biological Instruments methodology and equipment. The nucleotide sequence of the cDNA insert in p5bmi-1-ps is shown in SEQ ID NO:1 and the deduced amino acid sequence encoded by that sequence in SEQ ID NO:2.

### cDNA cloning of myo-inositol 1-phosphate synthase from immature seeds of LR33 soybeans

Seeds from the LR33 plants numbered 11, 16 and 20 and described in Example 3 (see Table 8) were harvested at about 50% through the seed filling period, removed from the pod and stored frozen at -80°C. For mRNA isolation, 2 g of frozen seed from the bulked seed population were ground in liquid nitrogen in a mortar and pestle.

The frozen powder was again ground in 10 mL of total RNA extraction buffer which consisted of 10 mM Tris-HCl, pH 9, 10 mM EDTA, 0.5% CTAB (cetyltrimethyl ammonium bromide), 0.8 M NaCl, 1% 2-mercaptoethanol, and 2% polyvinylpyrrolidone. Water for all reagents was treated with 0.05% diethylpyrocarbamate for 30 min then autoclaved. The tissue slurry was transferred to a 15 mL polypropylene tube and centrifuged at 5,500 x g for 15 min. The supernatant was passed through Miracloth (Calbiochem) into a second polypropylene tube and 0.3 volume of chloroform was added. The phases were separated by centrifugation at 5,500 x g for 10 min and the upper phase transferred to another polypropylene tube to which was added 1.5 volumes 10 mM Tris-HCl pH 9, 10 mM EDTA, 0.5% CTAB and 0.1% 2-mercaptoethanol. After a 30 min incubation at room temperature the nucleic acids were precipitated by centrifugation at 5,500 x g for 20 min.

The nucleic acids were re-dissolved in 0.4 mL of 1 M NaCl containing 0.1% 2-mercaptoethanol. After extraction with one volume of 1:1 phenol:chloroform, the nucleic acids were precipitated with two volumes of ethanol.

mRNA was purified from this nucleic acid fraction using the mRNA purification kit from Pharmacia. Approximately 12 µg of mRNA was obtained. Thirteen ng of the polyadenylated mRNA was used as template for amplification from oligo-dT using a GeneAmp® RNA-PCR kit (Perkin Elmer Cetus, part no. N808-0017). The reverse transcriptase reaction was run for 30 min at 42°C. For the PCR amplification, Vent™ DNA polymerase (New England Biolabs) was substituted for the DNA polymerase supplied by the kit manufacturer and an additional 2 µL of 100 mM magnesium sulfate was added to each 100 µL reaction. The 5' primer had the sequence shown in SEQ ID NO:3 and consists of bases 57 to 77 in SEQ ID NO:1 with the additional bases 5'-GGGAATTCCATATG-3' added to encode an Nde I site in the primer with eight additional 5' bases to enhance the restriction enzyme activity against the sequence.

The 3' primer had the sequence shown in SEQ ID NO:4 and consists of the reverse complement of bases 1566 to 1586 in SEQ ID NO:1 with the additional bases 5'-AAGGAAAAAAGCGGCCGC-3' added to provide a Not I site in the primer and ten additional bases to enhance restriction digestion. The PCR reaction was run for 35 cycles at a 52°C annealing temperature and 1.5 min extension time. A product of about 1550 base pairs was obtained and purified by passage through an Amicon 50 microfuge filter followed by extraction with an equal volume of 1:1 phenol:chloroform, extraction of the upper layer of the phenol:chloroform separation with one volume of chloroform and precipitation with ethanol. Five µg of the resulting, clean PCR product was digested overnight at 37°C with both Nde I and Not I. The restriction enzyme digest was de-proteinized by the above described phenol:chloroform extraction procedure and ligated into 2 µg of pET24aT7 expression vector (Novogen) that had also been digested with Nde I and Not I and treated with calf intestine alkaline phosphatase to hydrolyzed the terminal phosphates. The ligation mixture was used to transform electocompetant DH 10B *E. coli* cells and transformants were selected by growth on plates containing 30 mg 1⁻¹ kanamycin. Eighteen single colonies from the transformation plate were picked and placed in 100 µL of sterile water. Forty µL of the cell mix was used as the DNA template in a PCR reaction run with Taq™ polymerase (Perkin Elmer) using the primers and PCR conditions that were initially used to isolate the cDNA insert. Six colonies served as template to amplify a product of the correct size. The remaining 60 µL of the cell mix from these clones was grown in overnight culture and plasmid DNA preparations were made from each clone. The purified plasmid from each of the six clones was used to transform electrocompetant DE 3 *E. coli* cells.

### Functional expression of the myo-inositol-1-phosphate synthase from wildtype and LR33 soybeans in E coli

The wild type soybean *myo*-inositol 1-phosphate synthase was placed into the pET24aT7 expression vector by PCR amplification ofp5bmi-I-ps using the primers in SEQ ID NO:3 and SEQ ID NO:4, and the PCR amplification and cloning protocol described above for cloning the LR33 *myo*-inositol 1-phosphate synthase from reverse transcribed mRNA. In this case, plasmid preparations from nine DH 10B clones that were shown to contain plasmid with the cDNA insert were pooled and used to transform electrocompetant DE 3 *E. coli* cells.

Kanamycin resistant colonies were selected and six were chosen for inoculation of overnight cultures. The overnight cultures grown at 30°C in LB media with 30 mg 1⁻¹ kanamycin, were diluted two fold with fresh media, allowed to re-grow for 1 h, and induced by adding isopropyl-thiogalactoside to 1 mM final concentration.

Cells were harvested by centrifugation after 3 h and re-suspended in 50 µL of 50 mM Tris-HCl at pH 8.0 containing 0.1 mM DTT and 0.2 mM phenyl methylsulfonyl fluoride. A small amount of 1 mm glass beads were added and the mixture was sonicated three times for about five seconds each time with a microprobe sonicator. The mixture was centrifuged and the protein concentration of the supernatant was determined. One µg of protein from the soluble fraction of each clonal culture was separated by SDS-PAGE. Cultures which produced an addition protein band of about 60 kilodaltons in mass were chosen for activity assay.

For the assay, [³³P]glucose-6-phosphate was prepared by the hexokinase catalyzed phosphorylation of glucose using [³³P]-γ-ATP as the phosphate donor. After 30 min at room temperature, the reaction mix was passed through a SEP-PAC C 18 column (MilliPore Corp. Milford, MA) which had been washed first with 80% methanol and then water. The column pass through along with an additional 0.5 mL was collected and passed through a SEP-PAC SAX column which was then washed with 1 mL of 80% methanol. The [³³P]-glucose-6-phosphate was eluted with 2 mL of 0.02N HCl in 80% methanol. Forty µl of 1M Tris base was added and the methanol was removed under vacuum. The glucose-6-phosphate concentration of the remaining solution was determined by its conversion to 6-phosphoglucuronic acid by glucose-6-phosphate dehydrogenase. The NADPH produced in the reaction was quantitated by absorbance at 340 nm.

Ten µL of the cell extracts were incubated at 37°C for 30 min in 100 µL reactions that were 2 mM in glucose-6-phosphate (57,334 dpm total ³³P), 0.1 mM in NAD, and 15 mM in ammonium acetate. The reactions were heated to 90°C to precipitate protein, centrifuged to clear. Forty-seven µL of the supernatant was applied to a Dionex™ PA-1 column run at 0.9 ml min⁻¹ with 0.1 N NaOH and 0.1 N sodium acetate. Standards of *myo*-inositol 1-phosphate eluted from 8 through 10 min after injection and 1 min fractions of the separated reaction mixes were taken through that time range for scintillation counting. The radioactivity in the peak fractions was summed to obtain the conversion of glucose-6-phosphate to *myo*-inositol-1-phosphate and the results for one control *E. coli* DE 3 culture containing an empty pET24aT7 vector and two clones containing the soybean *myo*-inositol 1-phosphate synthase cDNA are shown in Table 12.

**Table 12**

| The specific activity (µmoles *myo*-inositol 1-phosphate produce min⁻¹ mg protein⁻¹) for three *E. coli* cell culture extracts (Soy Clones 1 and 2 contain the soybean *myo*-inositol 1-phosphate synthase while the control contains an empty plasmid.) | |
|---|---|
| LINE | SPECIFIC ACTIVITY |
| CONTROL | 0.024 µmol min⁻¹ mg⁻¹ |
| SOY CLONE 1 | 0.524 µmol min⁻¹ mg⁻¹ |
| SOY CLONE 2 | 0.892 µmol min⁻¹ mg⁻¹ |

In the assay as run, both cDNA-containing clones used essentially all of the available substrate and were therefore more than 35 fold more active than the control line. The soybean *myo*-inositol 1-phosphate synthase gene is therefore capable of producing a functional enzyme in *E. coli.*

The wild type soybean *myo*-inositol 1-phosphate synthase clone number 1 (Soy Clone 1) and three of the LR33 *myo*-inositol 1-phosphate synthase clones were grown for protein expression as described above. Five µg of protein from the soluble cell extract from each clone was separated by SDS-PAGE. LR33 clone number 10 (LR33-10) produced a soluble, 60 kilodalton protein in essentially the same abundance as did the wild type clone number 1. Four µg of protein from each of the two extracts was assayed for *myo*-inositol 1-phosphate synthase activity by the method described above using a 100 µL reaction that was 3 µm in NAD and 90 µm in glucose-6-phosphate. The specific activity of the wild type *myo*-inositol 1-phosphate synthase was 1.5 nmol min⁻¹ mg protein⁻¹ under these conditions while the specific activity of the LR33-derived *myo*-inositol 1-phosphate synthase was 0.16 nmol min⁻¹ mg protein⁻¹.

### The nucleotide sequence of the cDNA encoding the LR33 myo-inositol 1-phosphate synthase

The nucleotide sequence of the cDNA insert in clone LR33-10 (containing the nucleic acid fragment encoding the LR33 *myo*-inositol 1-phosphate synthase) was determined using the DH 10 B *E. coli* strain of that clone as the plasmid source and DNA sequencing as described for the wild type clone. The nucleotide sequence is shown in SEQ ID NO:5 and the deduced amino acid sequence obtained from the open reading frame of that clone in SEQ ID NO:6. SEQ ID NO:5 differs by single base pair change of G to T in the coding strand at base number 1241 from SEQ ID NO:1. That change results in a change of amino acid number 396 from lysine in the wild type sequence (SEQ ID NO:2) to asparagine in the LR33 amino acid sequence (SEQ ID NO:6).

To confirm that the base change resulted from a change in the LR33 genome rather than a PCR generated error which might have occurred during the cloning of the LR33 *myo*-inositol 1-phosphate synthase, two sets of PCR primers were prepared. The wild type primer (SEQ ID NO:7) and the LR33 primer (SEQ ID NO:8) were used to amplify genomic DNA prepared from dry seeds of soybean cultivar A2872 and from soybean line LR33 clone number 16 (see Table 8). The PCR primer described in SEQ ID NO:4 was used as the common antisense strand primer. At annealing temperatures of 62°C or 64°C and 35 cycles of annealing and extension, only the wild type primer produced a PCR product when A2872 DNA was used as a template and only the primer corresponding to the LR33 sequence produced a product when LR33 DNA was used as template.

To further check the specificity of the primers for detecting the mutation, DNA was prepared from six single plants grown from seed of the segregating LR33 clone number 7 line described in Example 3 (see Table 8). Out of six plants tested from the segregating population, two gave DNA that acted as template for both primers, three gave DNA that acted as a primer only with the wild type primer, and one gave DNA that produced a product only with the LR33 primer. These are the results expected from a population that contains heterozygotes containing one wild type and one mutant copy of the gene.

From the metabolite data and sequence data, we conclude that the observed seed phenotype of very low total raffinose saccharide sugars, very high sucrose and low phytic acid are all due to the single base change mutation described by the comparison of the wild type and LR33 sequences shown in SEQ ID NO:1 and SEQ ID NO:5.

### EXAMPLE 6

### TRANSFORMATION OF SOYBEANS TO ACHIEVE GENE SILENCING OF MYO-INOSITOL-1-PHOSPHATE-SYNTHASE AND THE ASSOCIATED SEED PHENOTYPE

### Construction of vectors for transformation of Glycine max for reduced expression of myo-inositol 1-phosphate synthase in developing soybean seeds

Plasmids containing the antisense or sense oriented soybean *myo*-inositol 1-phosphate synthase cDNA sequence under control of the soybean Kunitz Trypsin Inhibitor 3 (KTi3) promoter [Jofuku and Goldberg, (1989) *Plant Cell 1*:1079-1093], the *Phaseolus vulgaris* 7S seed storage protein (phaseolin) promoter [Sengupta-Gopalan et al., (1985) *Proc. Natl. Acad. Sci. USA 82*:3320-3324; Hoffman et al., (1988) *Plant Mol. Biol. 11*:717-729] and soybean β-conglycinin promoter [Beachy et al., (1985) *EMBO J. 4*:3047-3053], are constructed. The construction of vectors expressing the soybean *myo*-inositol-1-phosphate synthase cDNA under the control of these promoters is facilitated by the use of the following plasmids: pML70, pCW108 and pCW109A.

The pML70 vector contains the KTi3 promoter and the KTi3 3' untranslated region and was derived from the commercially available vector pTZ18R (Pharmacia) via the intermediate plasmids pML51, pML55, pML64 and pML65. A 2.4 kb Bst BI/Eco RI fragment of the complete soybean KTi3 gene [Jofuku and Goldberg *supra*], which contains all 2039 nucleotides of the 5' untranslated region and 390 bases of the coding sequence of the KTi3 gene ending at the Eco RI site corresponding to bases 755 to 761 of the sequence described in Jofuku et al., (1989) *Plant Cell 1*:427-435, was ligated into the Acc I/Eco RI sites of pTZ18R to create the plasmid pML51. The plasmid pML51 was cut with Nco I, filled in using Klenow, and religated, to destroy an Nco I site in the middle of the 5' untranslated region of the KTi3 insert, resulting in the plasmid pML55. The plasmid pML55 was partially digested with Xmn I/Eco RI to release a 0.42 kb fragment, corresponding to bases 732 to 755 of the above cited sequence, which was discarded. A synthetic Xmn I/Eco RI linker containing an Nco I site, was constructed by making a dimer of complementary synthetic oligonucleotides consisting of the coding sequence for an Xmn I site (5'-TCTTCC-3') and an Nco I site

(5'-CCATGGG-3')followed directly by part of an Eco RI site (5'-GAAGG-3'). The Xmn I and Nco I/Eco RI sites were linked by a short intervening sequence (5'-ATAGCCCCCCAA-3'). This synthetic linker was ligated into the Xmn I/Eco RI sites of the 4.94 kb fragment to create the plasmid pML64. The 3' untranslated region of the KTi3 gene was amplified from the sequence described in Jofuku et al., [*supra*] by standard PCR protocols (Perkin Elmer Cetus, GeneAmp PCR kit) using the primers ML51 and ML52. Primer ML51 contained the 20 nucleotides corresponding to bases 1072 to 1091 of the above cited sequence with the addition of nucleotides corresponding to Eco RV (5-'GATATC-3'), Nco I (5'-CCATGG-3'), Xba I (5'-TCTAGA-3'), Sma I (5'-CCCGGG-3') and Kpn I (5'-GGTACC-3') sites at the 5' end of the primer. Primer ML52 contained the exact complement of the nucleotides corresponding to bases 1242 to 1259 of the above cited sequence with the addition of nucleotides corresponding to Sma I (5'-CCCGGG-3'), Eco RI (5'-GAATTC-3'), Barn HI (5'-GGATCC-3') and Sal I (5'-GTCGAC-3') sites at the 5' end of the primer. The PCR-amplified 3' end of the KTi3 gene was ligated into the Nco I/Eco RI sites of pML64 to create the plasmid pML65. A synthetic multiple cloning site linker was constructed by making a dimer of complementary synthetic oligonucleotides consisting of the coding sequence for Pst I (5'-CTGCA-3'), Sal I (5'-GTCGAC-3'), Bam HI (5'-GGATCC-3') and Pst I (5'-CTGCA-3') sites. The linker was ligated into the Pst I site (directly 5' to the KTi3 promoter region) of pML65 to create the plasmid pML70.

The pCW108 vector contains the bean phaseolin promoter and 3' untranslated region and was derived from the commercially available pUC18 plasmid (Gibco-BRL) via plasmids AS3 and pCW104. Plasmid AS3 contains 495 base pairs of the bean *(Phaseolus vulgaris)* phaseolin (7S seed storage protein) promoter starting with 5'-TGGTCTTTTGGT-3' followed by the entire 1175 base pairs of the 3' untranslated region of the same gene [see sequence descriptions in Doyle et al., (1986) *J. Biol. Chem. 261*:9228-9238 and Slightom et al., (1983) *Proc. Natl. Acad. Sci. USA 80*:1897-1901; further sequence description may be found in World Patent Publication WO911/3993] cloned into the Hind III site of pUC18. The additional cloning sites of the pUC18 multiple cloning region (Eco RI, Sph I, Pst I and Sal I) were removed by digesting with Eco RI and Sal I, filling in the ends with Klenow and religating to yield the plasmid pCW104. A new multiple cloning site was created between the 495 bp of the 5' phaseolin and the 1175 bp of the 3' phaseolin by inserting a dimer of complementary synthetic oligonucleotides consisting of the coding sequence for a Nco I site (5'-CCATGG-3') followed by three filler bases (5'-TAG-3'), the coding sequence for a Sma I site (5'-CCCGGG-3'), the last three bases of a Kpn I site (5'-TAC-3'), a cytosine and the coding sequence for an Xba I site (5'-TCTAGA-3') to create the plasmid pCW108. This plasmid contains unique Nco I, Sma I, Kpn I and Xba I sites directly behind the phaseolin promoter.

The vector pCW109A contains the soybean β-conglycinin promoter sequence and the phaseolin 3' untranslated region and is a modified version of vector pCW109 which was derived from the commercially available plasmid pUC18 (Gibco-BRL). The vector pCW109 was made by inserting into the Hind III site of the cloning vector pUC18 a 555 bp 5' non-coding region (containing the promoter region) of the β-conglycinin gene followed by the multiple cloning sequence containing the restriction endonuclease sites for Nco I, Sma I, Kpn I and Xba I, as described for pCW108 above, then 1174 bp of the common bean phaseolin 3' untranslated region into the Hind III site (described above).

The β-conglycinin promoter region used is an allele of the published β-conglycinin gene [Doyle et al., (1986) *J. Biol. Chem. 261*:9228-9238] due to differences at 27 nucleotide positions. Further sequence description of this gene may be found in World Patent Publication W091/13993.

These three nucleic acid constructions constitute seed specific expression vectors with expression over a wide developmental period including the period of *myo*-inositol synthesis for subsequent conversion to phytic acid. Insertion of the sequences described in SEQ ID NO:1 and SEQ ID NO:5 into these vectors is facilitated by the PCR methods described in Example 5 above. PCR primers which are complementary to chosen regions of SED ID NO:1 or SEQ ID NO:5 may be synthesized with additional bases that constitute the recognition sequences of restriction endonucleases chosen from among those that also cut in the multiple cloning sequences following the promoter sequences of pML70, pCW108 and pCW109. Placement of the restriction sites may be chosen so as to direct the orientation of the nucleotide fragment from the soybean *myo*-inositol 1-phosphate synthase into the sense orientation to achieve either over expression or co-suppression or into the antisense orientation to achieve under expression.

### Transformation Of Somatic Soybean Embryo Cultures and Regeneration Of Soybean Plants

The following stock solutions and media are used to support the growth of soybean tissues *in vitro:*

| Stock Solutions: | | | |
|---|---|---|---|
| MS Sulfate (100X Stock) | | MS Halides (100X stock) | |
| | (g/L) | | (g/L) |
| MgSO₄ 7H₂O | 37.0 | CaCl₂ 2H₂O | 44.0 |
| MnSO₄ H₂O | 1.69 | KI | 0.083 |
| ZnSO₄ 7H₂O | 0.86 | CoCl₂ 6H₂O | 0.00125 |
| CuSO₄ 5H₂O | 0.0025 | KH₂PO₄ | 17.0 |
| | | H₃BO₃ | 0.63 |
| | | Na₂MoO₄ 2H₂O | 0.025 |

| B5 Vitamin | | MS FeEDTA (100X stock) | |
|---|---|---|---|
| | (g/L) | | (g/L) |
| *myo*-inositol | 10.0 | Na₂EDTA | 3.72 |
| nicotinic acid | 0.10 | FeSO₄ 7H₂O | 2.784 |
| pyridoxine HCl | 0.10 | | |

| Media: | | | | |
|---|---|---|---|---|
| Component (per liter) | SB55 | SBP6 | SB103 | SB71-1 |
| MS Sulfate Stock | 10 mL | 10 mL | 10 mL | - |
| MS Halides Stock | 10 mL | 10 mL | 10 mL | - |
| MS FeEDTA Stock | 10 mL | 10 mL | 10 mL | - |
| B5 Vitamin Stock | 1 mL | 1 mL | - | 1 mL |
| 2,4-D Stock (10 mg/ml) | 1 mL | 0.5 mL | - | - |
| Sucrose | 60 g | 60 g | - | 3% |
| Maltose | - | - | 6% | - |
| Asparagine | 0.667 g | 0.667 g | - | - |
| NH₄NO | 0.8 g | 0.8 g | - | - |
| KNO₃ | 3.033g | 3.033g | - | - |
| MgCl₂ | - | - | 750 mg | 750 mg |
| Gelrite | - | - | 0.2% | 0.2% |
| pH | 5.7 | 5.7 | 5.7 | 5.7 |

Soybean embryogenic suspension cultures are maintained in 35 mL liquid media (SB55 or SBP6) on a rotary shaker, 150 rpm, at 28°C with mixed florescent and incandescent lights on a 16:8 h day/night schedule. Cultures are subcultured every four weeks by inoculating approximately 35 mg of tissue into 35 mL of liquid medium.

Soybean embryogenic suspension cultures may be transformed with seed specific expression vectors containing either the sense or antisense oriented *myo*-inositol 1-phosphate synthase by the method of particle gun bombardment (see Kline et al. (1987) *Nature (London) 327*:70). A DuPont Biolistic™ PDS1000/HE instrument (helium retrofit) is used for these transformations.

To 50 mL of a 60 mg/mL 1 µm gold particle suspension are added (in order): 5 µL DNA (1 µg/µL), 20 µL spermidine (0.1 M), and 50 µl CaCl₂ (2.5 M). The particle preparation is agitated for 3 min, spun in a microfuge for 10 sec and the supernatant removed. The DNA-coated particles are then washed once in 400 µL 70% ethanol and resuspended in 40 µL of anhydrous ethanol. The DNA/particle suspension is sonicated three times for 1 sec each. Five µL of the DNA-coated gold particles are then loaded on each macro carrier disk.

Approximately 300-400 mg of a four week old suspension culture is placed in an empty 60 x 15 mm petri dish and the residual liquid removed from the tissue with a pipette. For each transformation experiment, approximately 5-10 plates of tissue were normally bombarded. Membrane rupture pressure is set at 1000 psi and the chamber is evacuated to a vacuum of 28 inches of mercury. The tissue is placed approximately 3.5 inches away from the retaining screen and bombarded three times. Following bombardment, the tissue is placed back into liquid and cultured as described above.

Eleven days post bombardment, the liquid media is exchanged with fresh SB55 containing 50 mg/mL hygromycin. Thereafter, the selective media is refreshed weekly. Seven weeks post bombardment, green, transformed tissue may be observed growing from untransformed, necrotic embryogenic clusters. Isolated green tissue is removed and inoculated into individual flasks to generate new, clonally propagated, transformed embryogenic suspension cultures. Thus each new line is treated as independent transformation event. These suspensions can then be maintained as suspensions of embryos clustered in an immature developmental stage through subculture or regenerated into whole plants by maturation and germination of individual somatic embryos.

Transformed embryogenic clusters are removed from liquid culture and placed on a solid agar media (SB103) containing no hormones or antibiotics. Embryos are cultured for eight weeks at 26°C with mixed florescent and incandescent lights on a 16:8 h day/night schedule. During this period, individual embryos can be removed from the clusters and analyzed at various stages of embryo development. After eight weeks somatic embryos become suitable for germination. For germination, eight week old embryos are removed from the maturation medium and dried in empty petri dishes for 1 to 5 days. The dried embryos are then planted in SB71-1 medium were they are allowed to germinate under the same lighting and germination conditions described above. Germinated embryos can then transferred to sterile soil and grown to maturity for seed collection.

While in the globular embryo state in liquid culture as described above, somatic soybean embryos contain very low amounts of triacylglycerol or storage proteins typical of maturing, zygotic soybean embryos. At this developmental stage, the ratio of total triacylglyceride to total polar lipid (phospholipids and glycolipid) is about 1:4, as is typical of zygotic soybean embryos at the developmental stage from which the somatic embryo culture was initiated. At the globular stage as well, the mRNAs for the prominent seed proteins (α'-subunit of β-conglycinin, Kunitz Trypsin Inhibitor 3 and Soybean Seed Lectin) are essentially absent. Upon transfer to hormone-free media to allow differentiation to the maturing somatic embryo state as described above, triacylglycerol becomes the most abundant lipid class. As well, mRNAs for α'-subunit of β-conglycinin, Kunitz Trypsin Inhibitor 3 and Soybean Seed Lectin become very abundant messages in the total mRNA population. In these respects the somatic soybean embryo system behaves very similarly to maturing zygotic soybean *embryos in vivo.* During the early maturation period the main soluble carbohydrates present in the somatic embryos are sucrose, glucose and maltose (the supplied sugar during the maturation phase). As the somatic embryos mature and begin to yellow, both raffinose and stachyose are formed. In this respect as well the phenotype of the somatic embryos is very similar to zygotic embryos as they go through the late stages of seed development. Thus selection for embryos that are transformed with seed specific expression vectors which direct the expression of the nucleotide sequences described in SEQ ID NO:1 or SEQ ID NO:5 I in either the sense or antisense orientation and that produce reduced amount of raffinose and stachyose should lead to the regeneration of mature, fertile soybean plants which bear seeds with that same phenotype.

## Claims

1. A soybean plant homozygous for at least one gene encoding a mutant *myo*-inositol 1-phosphate synthase, the gene having a mutation in the sequence of SEQ ID NO: 1, the mutant *myo*-inositol 1-phosphate synthase having reduced enzymatic activity when compared to a wild type *myo*-inositol 1-phosphate synthase, the gene conferring a heritable phenotype of (i) a seed phytic acid content of less than 17 µmol/g, (ii) a seed content of raffinose plus stachyose combined of less than 14.5 µmol/g, and (iii) a seed sucrose content of greater than 200 µmol/g, provided that the plant is not LR33 (deposited at the ATCC under accession No. 97988).

2. A soybean plant as claimed in claim 1 wherein the gene has the sequence of SEQ ID NO: 5 or the gene encodes a mutant *myo*-inositol 1-phosphate synthase of SEQ ID NO: 6.

3. A soybean plant having the phenotype of the soybean plant of claim 1 comprising a chimeric gene selected from the group consisting of:
(i) a chimeric gene comprising an isolated nucleic acid fragment encoding a soybean *myo*-inositol-1-phosphate synthase of SEQ ID NO: 2 or the complement of the nucleic acid fragment encoding a soybean *myo*-inositol-1-phosphate synthase of SEQ ID NO: 2, operably linked to suitable regulatory sequences, wherein expression of the chimeric gene results in reduced enzymatic activity of a native gene encoding a soybean *myo*-inositol-1-phosphate synthase when compared to a soybean not having the chimeric gene; and
(ii) a chimeric gene comprising a subfragment of an isolated nucleic acid fragment encoding a soybean *myo*-inositol-1-phosphate synthase of SEQ ID NO: 2 or the complement of a subfragment of an isolated nucleic acid fragment encoding a soybean *myo*-inositol-1-phosphate synthase of SEQ ID NO: 2, wherein expression of the chimeric gene results in reduced enzymatic activity of a native gene encoding a soybean *myo*-inositol-1-phosphate synthase when compared to a soybean not having the chimeric gene.

4. Seeds of the soybean plant of any one of claims 1 to 3 wherein said seeds contain (i) a seed phytic acid content of less than 17 µmol/g (ii) a seed content of raffinose plus stachyose combined of less than 14.5 µmol/g and (iii) a seed sucrose content of greater than 200 µmol/g.

5. A method for producing a soy protein product comprising processing the seeds of claim 4 to obtain the desired soybean protein product.

6. A method for producing a soy protein product obtainable from seeds of a soybean plant homozygous for at least one gene encoding a mutant *myo*-inositol 1-phosphate synthase having reduced enzymatic activity when compared to a wild type *myo*-inositol 1-phosphate synthase, the gene conferring a heritable phenotype of
(i) a seed phytic acid content of less than 17 µmol/g,
(ii) a seed content of raffinose plus stachyose combined of less than 14.5 µmol/g, and (iii) a seed sucrose content of greater than 200 µmol/g comprising:
(a) crossing an agronomically elite soybean plant with LR33 (deposited at the ATCC under accession No. 97988) or any of the soybean plants of claim 1 or 3,
(b) screening the seed of progeny plants obtained from step (a) for (i) a seed phytic acid content less than 17 µmol/g, (ii) a seed content of raffinose plus stachyose combined of less than 14.5 µmol/g, and (iii) a seed sucrose content of greater than 200 µmol/g; and
(c) processing the seed selected in step (b) to obtain the desired soybean protein product.

## Patentansprüche

1. Sojabohnenpflanze, homozygot für mindestens ein Gen, das eine mutierte *myo*-Inositol-1-phosphat-Synthase codiert, wobei das Gen eine Mutation in der Sequenz von SEQ ID NO: 1 aufweist, wobei die mutierte *myo*-Inositol-1-phosphat-Synthase im Vergleich zu einer *myo*-Inositol-1-phosphat-Synthase des Wildtyps eine reduzierte enzymatische Aktivität aufweist, wobei das Gen einen erblichen Phänotyp von (i) einem Phytinsäuregehalt im Samen von weniger als 17 µmol/g, (ii) einem kombinierten Raffinose- plus Stachyose-Gehalt im Samen von weniger als 14,5 µmol/g und (iii) einem Saccharose-Gehalt im Samen von größer als 200 µmol/g verleiht, vorausgesetzt dass die Pflanze nicht LR33 (hinterlegt bei der ATCC unter Zugangs-Nr. 97988) darstellt.

2. Sojabohnenpflanze nach Anspruch 1, worin das Gen die Sequenz von SEQ ID NO: 5 aufweist oder das Gen eine mutierte *myo*-Inositol-1-phosphat-Synthase von SEQ ID NO: 6 codiert.

3. Sojabohnenpflanze, die den Phänotyp der Sojabohnenpflanze nach Anspruch 1 aufweist, umfassend ein chimäres Gen, das aus der Gruppe ausgewählt ist, bestehend aus:
(i) Einem chimären Gen, das ein isoliertes Nukleinsäurefragment umfasst, codierend eine *myo*-Inositol-1-phosphat-Synthase von SEQ ID NO: 2 der Sojabohne oder das Komplement des Nukleinsäurefragments, codierend eine *myo*-Inositol-1-phosphat-Synthase von SEQ ID NO: 2 der Sojabohne, funktionsfähig verknüpft mit geeigneten Regulationssequenzen, worin die Expression des chimären Gens, im Vergleich zu einer Sojabohne, die kein chimäres Gen aufweist, in reduzierter enzymatischer Aktivität eines nativen Gens, codierend eine *myo*-Insitol-1-phosphat-Synthase der Sojabohne, resultiert; und
(ii) einem chimären Gen, das ein Subfragment eines isolierten Nukleinsäurefragments umfasst, codierend eine *myo*-Inositol-1-phosphat-Synthase von SEQ ID NO: 2 der Sojabohne oder das Komplement eines Subfragments eines isolierten Nukleinsäurefragments, codierend eine *myo*-Inositol-1-phosphat-Synthase von SEQ ID NO: 2 der Sojabohne, worin die Expression des chimären Gens, im Vergleich zu einer Sojabohne, die kein chimäres Gen aufweist, in reduzierter enzymatischer Aktivität eines nativen Gens, codierend eine *myo*-Inositol-1-phosphat-Synthase der Sojabohne, resultiert.

4. Samen der Sojabohnenpflanze nach einem der Ansprüche 1 bis 3, worin genannter Samen (i) einen Phytinsäuregehalt im Samen von weniger als 17 µmol/g, (ii) einen kombinierten Raffinose- plus Stachyosegehalt im Samen von weniger als 14,5 µmol/g und (iii) einen Saccharosegehalt im Samen von größer als 200 µmol/g enthält.

5. Verfahren zur Herstellung eines Sojaprotein-Produkts, umfassend die Verarbeitung der Samen nach Anspruch 4, um das gewünschte Sojabohnen-Proteinprodukt zu erhalten.

6. Verfahren zur Herstellung eines Sojaprotein-Produktes, das aus Samen einer Sojabohnenpflanze, homozygot für mindestens ein Gen, das eine mutierte *myo*-Inositol-1-phosphat-Synthase codiert, die im Vergleich zu einer *myo*-Inositol-1-phosphat-Synthase des Wildtyps eine reduzierte enzymatische Aktivität aufweist, erhältlich ist, wobei das Gen einen erblichen Phänotyp von (i) einem Phytinsäuregehalt im Samen von weniger als 17 µmol/g, (ii) einem kombinierten Raffinose- plus Stachyosegehalt im Samen von weniger als 14,5 µmol/g und (iii) einem Saccharosegehalt im Samen von größer als 200 µmol/g verleiht, umfassend:
(a) Kreuzen einer agronomisch elitären Sojabohnenpflanze mit LR33 (hinterlegt bei der ATCC unter Zugangs-Nr. 97988) oder jedweder der Sojabohnenpflanzen nach Anspruch 1 oder 3;
(b) Screening des Samens von der aus Schritt (a) erhaltenen Pflanzennachkommenschaft auf (i) einen Phytinsäuregehalt im Samen von weniger als 17 µmol/g, (ii) einen kombinierten Raffinose- plus Stachyosegehalt im Samen von weniger als 14,5 µmol/g und (iii) einen Saccharosegehalt von größer als 200 µmol/g; und
(c) Verarbeitung des in Schritt (b) ausgewählten Samens, um das gewünschte Sojaprotein-Produkt zu erhalten.

## Revendications

1. Plante de soja homozygote pour au moins un gène codant une *myo*-inositol-1-phosphate-synthase mutante, le gène ayant une mutation dans la séquence de la SEQ ID NO:1, la *myo*-inositol-1-phosphate-synthase mutante ayant une activité enzymatique réduite lorsque comparée à une *myo*-inositol-1-phosphate-synthase de type sauvage, le gène conférant un phénotype héritable (i) d'une teneur de graines en acide phytique de moins de 17 µmol/g, (ii) d'une teneur de graines en raffinose plus stachyose combinés de moins de 14,5 µmol/g, et (iii) d'une teneur de graines en saccharose de plus de 200 µmol/g, à condition que la plante ne soit pas LR33 (déposé auprès de la ATCC sous le n° d'accès 97988).

2. Plante de soja telle que revendiquée dans la revendication 1, dans laquelle le gène a la séquence de la SEQ ID NO:5 ou bien le gène code une *myo*-inositol-1-phosphate-synthase mutante de la SEQ ID NO:6.

3. Plante de soja ayant le phénotype de la plante de soja de la revendication 1, comprenant un gène chimérique sélectionné parmi le groupe consistant en:
(i) un gène chimérique comprenant un fragment d'acide nucléique isolé codant une *myo*-inositol-1-phosphate-synthase de soja de la SEQ ID NO:2 ou le complément du fragment d'acide nucléique codant une *myo*-inositol-1-phosphate-synthase de soja de la SEQ ID NO:2, lié de manière opérationnelle à des séquences régulatrices appropriées, où l'expression du gène chimérique résulte en une activité enzymatique réduite d'un gène natif codant une *myo*-inositol-1-phosphate-synthase de soja lorsque comparé à du soja n'ayant pas le gène chimérique; et
(ii) un gène chimérique comprenant un sous-fragment d'un fragment d'acide nucléique isolé codant une *myo*-inositol-1-phosphate-synthase de soja de la SEQ ID NO:2 ou le complément d'un sous-fragment d'un fragment d'acide nucléique isolé codant une *myo*-inositol-1-phosphate-synthase de soja de la SEQ ID NO:2, où l'expression du gène chimérique résulte en une activité enzymatique réduite d'un gène natif codant une *myo*-inositol-1-phosphate-synthase de soja comparé à du soja n'ayant pas le gène chimérique.

4. Graines de la plante de soja de l'une quelconque des revendications 1 à 3, où lesdites graines contiennent (i) une teneur de graines en acide phytique de moins de 17 µmol/g, (ii) une teneur de graines en raffinose plus stachyose combinés de moins de 14,5 µmol/g et (iii) une teneur de graines en saccharose de plus de 200 µmol/g.

5. Procédé de production d'un produit protéique de soja comprenant traiter les graines de la revendication 4 pour obtenir le produit protéique de soja désiré.

6. Procédé de production d'un produit protéique de soja pouvant être obtenu de graines d'une plante de soja homozygote pour au moins un gène codant une *myo*-inositol-1-phosphate-synthase mutante ayant une activité enzymatique réduite lorsque comparée à une *myo*-inositol-1-phosphate-synthase de type sauvage, le gène conférant un phénotype héritable (i) d'une teneur de graines en acide phytique de moins de 17 µmol/g, (ii) d'une teneur de graines en raffinose plus stachyose combinés de moins de 14,5 µmol/g, et (iii) d'une teneur de graines en saccharose de plus de 200 µmol/g, comprenant:
(a) croiser une plante de soja agronomiquement d'élite avec LR33 (déposé auprès de ATCC sous le n° d'accès 97988) ou l'une quelconque des plantes de soja de la revendication 1 ou 3;
(b) cribler les graines des descendants des plantes obtenus de l'étape (a) pour (i) une teneur de graines en acide phytique de moins de 17 µmol/g, (ii) une teneur de graines en raffinose plus stachyose combinés de moins de 14,5 µmol/g, et (iii) une teneur de graines en saccharose de plus de 200 µmol/g; et
(c) traiter les graines sélectionnées à l'étape (b) pour obtenir le produit protéique de soja désiré.


### SEQUENCE LISTING

(1) GENERAL INFORMATION:
   (i) APPLICANT: HITZ, WILLIAM D.
      SEBASTIAN, SCOTT ANTHONY
   (ii) TITLE OF INVENTION: SOYBEAN PLANTS PRODUCING SEEDS WITH
      REDUCED LEVELS OF RAFFINOSE
      SACCHARIDES AND PHYTIC ACID
   (iii) NUMBER OF SEQUENCES: 8
   (iv) CORRESPONDENCE ADDRESS:
      (A) ADDRESSEE: E. I. DU PONT DE NEMOURS AND COMPANY
      (B) STREET: 1007 MARKET STREET
      (C) CITY: WILMINGTON
      (D) STATE: DELAWARE
      (E) COUNTRY: UNITED STATES OF AMERICA
      (F) ZIP: 19898
   (v) COMPUTER READABLE FORM:
      (A) MEDIUM TYPE: DISKETTE, 3.50 INCH
      (B) COMPUTER: IBM PC COMPATIBLE
      (C) OPERATING SYSTEM: MICROSOFT WINDOWS 95
      (D) SOFTWARE: MICROSOFT WORD FOR WINDOWS 95 (7.0)
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: MAJARIAN, WILLIAM R.
      (B) REGISTRATION NUMBER: P41,173
      (C) REFERENCE/DOCKET NUMBER: BB-1077
   (ix) TELECOMMUNICATION INFORMATION:
      (A) TELEPHONE: (302)992-4926
      (B) TELEFAX: (302)773-0164
(2) INFORMATION FOR SEQ ID NO:1:
   (i) SEQUENCE CHARACTERISTICS:
      (A) LENGTH: 1782 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Soybean line LR13
   (vii) IMMEDIATE SOURCE:
      (B) CLONE: p5bmi-1-ps
   (ix) FEATURE:
      (A) NAME/KEY: CDS
      (B) LOCATION: 54..1586
   (xi) SEQUENCE DESCRIPTION: SEQ ID NO:1:
   CTCTTCTTTA TTCCTTTTGT AATTTCATTC ATTCTTAATC TTTGTGAAAA ATAATGTTCA    60
   TCGAGAATTT TAAGGTTGAG TGTCCTAATG TGAAGTACAC CGAGACTGAG ATTCAGTCCG   120
   TGTACAACTA CGAAACCACC GAACTTGTTC ACGAGAACAG GAATGGCACC TATCAGTGGA   180
   TTGTCAAACC CAAATCTGTC AAATACGAAT TTAAAACCAA CATCCATGTT CCTAAATTAG   240
   GGGTAATGCT TGTGGGTTGG GGTGGAAACA ACGGCTCAAC CCTCACCGGT GGTGTTATTG   300
   CTAACCGAGA GGGCATTTCA TGGGCTACAA AGGACAAGAT TCAACAAGCC AATTACTTTG   360
   GCTCCCTCAC CCAAGCCTCA GCTATCCGAG TTGGGTCCTT CCAGGGAGAG GAAATCTATG   420
   CCCCATTCAA GAGCCTGCTT CCAATGGTTA ACCCTGACGA CATTGTGTTT GGGGGATGGG   480
   ATATCAGCAA CATGAACCTG GCTGATGCCA TGGCCAGGGC AAAGGTGTTT GACATCGATT   540
   TGCAGAAGCA GTTGAGGCCT TACATGGAAT CCATGCTTCC ACTCCCCGGA ATCTATGACC   600
   CGGATTTCAT TGCTGCCAAC CAAGAGGAGC GTGCCAACAA CGTCATCAAG GGCACAAAGC   660
   AAGAGCAAGT TCAACAAATC ATCAAAGACA TCAAGGCGTT TAAGGAAGCC ACCAAAGTGG   720
   ACAAGGTGGT TGTACTGTGG ACTGCCAACA CAGAGAGGTA CAGTAATTTG GTTGTGGGCC   780
   TTAATGACAC CATGGAGAAT CTCTTGGCTG CTGTGGACAG AAATGAGGCT GAGATTTCTC   840
   CTTCCACCTT GTATGCCATT GCTTGTGTTA TGGAAAATGT TCCTTTCATT AATGGAAGCC   900
   CTCAGAACAC TTTTGTACCA GGGCTGATTG ATCTTGCCAT CGCGAGGAAC ACTTTGATTG   960
   GTGGAGATGA CTTCAAGAGT GGTCAGACCA AAATGAAATC TGTGTTGGTT GATTTCCTTG   1020
   TGGGGGCTGG TATCAAGCCA ACATCTATAG TCAGTTACAA CCATCTGGGA AACAATGATG   1080
   GTATGAATCT TTCGGCTCCA CAAACTTTCC GTTCCAAGGA AATCTCCAAG AGCAACGTTG   1140
   TTGATGATAT GGTCAACAGC AATGCCATCC TCTATGAGCC TGGTGAACAT CCAGACCATG   1200
   TTGTTGTTAT TAAGTATGTG CCTTACGTAG GGGACAGCAA GAGAGCCATG GATGAGTACA   1260
   CTTCAGAGAT ATTCATGGGT GGAAAGAGCA CCATTGTTTT GCACAACACA TGCGAGGATT   1320
   CCCTCTTAGC TGCTCCTATT ATCTTGGACT TGGTCCTTCT TGCTGAGCTC AGCACTAGAA   1380
   TCGAGTTTAA AGCTGAAAAT GAGGGAAAAT TCCACTCATT CCACCCAGTT GCTACCATCC   1440
   TCAGCTACCT CACCAAGGCT CCTCTGGTTC CACCGGGTAC ACCAGTGGTG AATGCATTGT   1500
   CAAAGCAGCG TGCAATGCTG GAAAACATAA TGAGGGCTTG TGTTGGATTG GCCCCAGAGA   1560
   ATAACATGAT TCTCGAGTAC AAGTGAAGCA TGGGACCGAA GAATAATATA GTTGGGGTAG   1620
   CCTAGCTGAA TGTTTTATGT TAATAATATG TTTGCTTATA ATTTTGCAAG TGTAATTGAA   1680
   TGCATCAGCT TCATTAATGC TTTAGAGCGG GGCATATTCT GTTTACTAGG AACATGAATG   1740
   AATGTAGTAT AATTTTGTGT AAAAAAAAAA AAAAAAAAAA AA                      1782
(2) INFORMATION FOR SEQ ID NO:2:
   (i) SEQUENCE CHARACTERISTICS:
      (A) LENGTH: 510 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Phe Ile Glu Asn Phe Lys Val Glu Cys Pro Asn Val Lys Tyr Thr
   1               5                   10                  15
   Glu Thr Glu Ile Gln Ser Val Tyr Asn Tyr Glu Thr Thr Glu Leu Val
               20                  25                  30
   His Glu Asn Arg Asn Gly Thr Tyr Gln Trp Ile Val Lys Pro Lys Ser
           35                  40                  45
   Val Lys Tyr Glu Phe Lys Thr Asn Ile His Val Pro Lys Leu Gly Val
       50                  55                  60
   Met Leu Val Gly Trp Gly Gly Asn Asn Gly Ser Thr Leu Thr Gly Gly
   65                  70                  75                  80
   Val Ile Ala Asn Arg Glu Gly Ile Ser Trp Ala Thr Lys Asp Lys Ile
                   85                  90                  95
   Gln Gln Ala Asn Tyr Phe Gly Ser Leu Thr Gln Ala Ser Ala Ile Arg
   100 105 110
   Val Gly Ser Phe Gln Gly Glu Glu Ile Tyr Ala Pro Phe Lys Ser Leu
   115 120 125
   Leu Pro Met Val Asn Pro Asp Asp Ile Val Phe Gly Gly Trp Asp Ile
   130 135 140
   Ser Asn Met Asn Leu Ala Asp Ala Met Ala Arg Ala Lys Val Phe Asp
   145 150 155 160
   Ile Asp Leu Gln Lys Gln Leu Arg Pro Tyr Met Glu Ser Met Leu Pro
   165 170 175
   Leu Pro Gly Ile Tyr Asp Pro Asp Phe Ile Ala Ala Asn Gln Glu Glu
   180 185 190
   Arg Ala Asn Asn Val Ile Lys Gly Thr Lys Gln Glu Gln Val Gln Gln
   195 200 205
   Ile Ile Lys Asp Ile Lys Ala Phe Lys Glu Ala Thr Lys Val Asp Lys
   210 215 220
   Val Val Val Leu Trp Thr Ala Asn Thr Glu Arg Tyr Ser Asn Leu Val
   225 230 235 240
   Val Gly Leu Asn Asp Thr Met Glu Asn Leu Leu Ala Ala Val Asp Arg
   245 250 255
   Asn Glu Ala Glu Ile Ser Pro Ser Thr Leu Tyr Ala Ile Ala Cys Val
   260 265 270
   Met Glu Asn Val Pro Phe Ile Asn Gly Ser Pro Gln Asn Thr Phe Val
   275 280 285
   Pro Gly Leu Ile Asp Leu Ala Ile Ala Arg Asn Thr Leu Ile Gly Gly
   290 295 300
   Asp Asp Phe Lys Ser Gly Gln Thr Lys Met Lys Ser Val Leu Val Asp
   305 310 315 320
   Phe Leu Val Gly Ala Gly Ile Lys Pro Thr Ser Ile Val Ser Tyr Asn
   325 330 335
   His Leu Gly Asn Asn Asp Gly Met Asn Leu Ser Ala Pro Gln Thr Phe
   340 345 350
   Arg Ser Lys Glu Ile Ser Lys Ser Asn Val Val Asp Asp Met Val Asn
   355 360 365
   Ser Asn Ala Ile Leu Tyr Glu Pro Gly Glu His Pro Asp His Val Val
   370 375 380
   Val Ile Lys Tyr Val Pro Tyr Val Gly Asp Ser Lys Arg Ala Met Asp
   385 390 395 400
   Glu Tyr Thr Ser Glu Ile Phe Met Gly Gly Lys Ser Thr Ile Val Leu
                   405                 410                 415
   His Asn Thr Cys Glu Asp Ser Leu Leu Ala Ala Pro Ile Ile Leu Asp
               420                 425                 430
   Leu Val Leu Leu Ala Glu Leu Ser Thr Arg Ile Glu Phe Lys Ala Glu
           435                 440                 445
   Asn Glu Gly Lys Phe His Ser Phe His Pro Val Ala Thr Ile Leu Ser
       450                 455                 460
   Tyr Leu Thr Lys Ala Pro Leu Val Pro Pro Gly Thr Pro Val Val Asn
   465                 470                 475                 480
   Ala Leu Ser Lys Gln Arg Ala Met Leu Glu Asn Ile Met Arg Ala Cys
                   485                 490                 495
   Val Gly Leu Ala Pro Glu Asn Asn Met Ile Leu Glu Tyr Lys
               500                 505                 510
(2) INFORMATION FOR SEQ ID NO:3:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: DNA (oligonucleotide)
   (xi) SEQUENCE DESCRIPTION: SEQ ID NO:3:
   GGGAATTCCA TATGTTCATC GAGAATTTTA AGGTT                    35
(2) INFORMATION FOR SEQ ID NO:4:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (ii) MOLECULE TYPE: DNA (oligonucleotide)
   (xi) SEQUENCE DESCRIPTION: SEQ ID NO:4:
   AAGGAAAAAA GCGGCCGCTC ACTTGTACTC GAGAATCAT                39
(2) INFORMATION FOR SEQ ID NO:5:
   (i) SEQUENCE CHARACTERISTICS:
      (A) LENGTH: 1775 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Soybean line LR33
   (vii) IMMEDIATE SOURCE:
      (B) CLONE: LR33-10
   (ix) FEATURE:
      (A) NAME/KEY: CDS
      (B) LOCATION: 1..1533
   (ix) FEATURE:
      (A) NAME/KEY: POINT MUTATION
      (B) LOCATION: 1188
   (xi) SEQUENCE DESCRIPTION: SEQ ID NO:5:
(2) INFORMATION FOR SEQ ID NO:6:
   (i) SEQUENCE CHARACTERISTICS:
      (A) LENGTH: 510 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:6:
   Met Phe Ile Glu Asn Phe Lys Val Glu Cys Pro Asn Val Lys Tyr Thr
   1               5                   10                  15
   Glu Thr Glu Ile Gln Ser Val Tyr Asn Tyr Glu Thr Thr Glu Leu Val
               20                  25                  30
   His Glu Asn Arg Asn Gly Thr Tyr Gln Trp Ile Val Lys Pro Lys Ser
           35                  40                  45
   Val Lys Tyr Glu Phe Lys Thr Asn Ile His Val Pro Lys Leu Gly Val
       50                  55                  60
   Met Leu Val Gly Trp Gly Gly Asn Asn Gly Ser Thr Leu Thr Gly Gly
   65                  70                  75                  80
   Val Ile Ala Asn Arg Glu Gly Ile Ser Trp Ala Thr Lys Asp Lys Ile
                   85                  90                  95
   Gln Gln Ala Asn Tyr Phe Gly Ser Leu Thr Gln Ala Ser Ala Ile Arg
               100                 105                 110
   Val Gly Ser Phe Gln Gly Glu Glu Ile Tyr Ala Pro Phe Lys Ser Leu
           115                 120                 125
   Leu Pro Met Val Asn Pro Asp Asp Ile Val Phe Gly Gly Trp Asp Ile
       130                 135                 140
   Ser Asn Met Asn Leu Ala Asp Ala Met Ala Arg Ala Lys Val Phe Asp
   145                 150                 155                 160
   Ile Asp Leu Gln Lys Gln Leu Arg Pro Tyr Met Glu Ser Met Leu Pro
   165 170 175
   Leu Pro Gly Ile Tyr Asp Pro Asp Phe Ile Ala Ala Asn Gln Glu Glu
   180 185 190
   Arg Ala Asn Asn Val Ile Lys Gly Thr Lys Gln Glu Gln Val Gln Gln
   195 200 205
   Ile Ile Lys Asp Ile Lys Ala Phe Lys Glu Ala Thr Lys Val Asp Lys
   210 215 220
   Val Val Val Leu Trp Thr Ala Asn Thr Glu Arg Tyr Ser Asn Leu Val
   225 230 235 240
   Val Gly Leu Asn Asp Thr Met Glu Asn Leu Leu Ala Ala Val Asp Arg
   245 250 255
   Asn Glu Ala Glu Ile Ser Pro Ser Thr Leu Tyr Ala Ile Ala Cys Val
   260 265 270
   Met Glu Asn Val Pro Phe Ile Asn Gly Ser Pro Gln Asn Thr Phe Val
   275 280 285
   Pro Gly Leu Ile Asp Leu Ala Ile Ala Arg Asn Thr Leu Ile Gly Gly
   290 295 300
   Asp Asp Phe Lys Ser Gly Gln Thr Lys Met Lys Ser Val Leu Val Asp
   305 310 315 320
   Phe Leu Val Gly Ala Gly Ile Lys Pro Thr Ser Ile Val Ser Tyr Asn
   325 330 335
   His Leu Gly Asn Asn Asp Gly Met Asn Leu Ser Ala Pro Gln Thr Phe
   340 345 350
   Arg Ser Lys Glu Ile Ser Lys Ser Asn Val Val Asp Asp Met Val Asn
   355 360 365
   Ser Asn Ala Ile Leu Tyr Glu Pro Gly Glu His Pro Asp His Val Val
   370 375 380
   Val Ile Lys Tyr Val Pro Tyr Val Gly Asp Ser Asn Arg Ala Met Asp
   385 390 395 400
   Glu Tyr Thr Ser Glu Ile Phe Met Gly Gly Lys Ser Thr Ile Val Leu
   405 410 415
   His Asn Thr Cys Glu Asp Ser Leu Leu Ala Ala Pro Ile Ile Leu Asp
   420 425 430
   Leu Val Leu Leu Ala Glu Leu Ser Thr Arg Ile Glu Phe Lys Ala Glu
   435 440 445
   Asn Glu Gly Lys Phe His Ser Phe His Pro Val Ala Thr Ile Leu Ser
   450 455 460
   Tyr Leu Thr Lys Ala Pro Leu Val Pro Pro Gly Thr Pro Val Val Asn
   465                 470                 475                 480
   Ala Leu Ser Lys Gln Arg Ala Met Leu Glu Asn Ile Met Arg Ala Cys
                   485                 490                 495
   Val Gly Leu Ala Pro Glu Asn Asn Met Ile Leu Glu Tyr Lys
               500                 505                 510
(2) INFORMATION FOR SEQ ID NO:7:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: Synthetic ogligonucleotide
   (xi) SEQUENCE DESCRIPTION: SEQ ID NO:7:
   CGTAGGGGAC AGCAAG                                                  16
(2) INFORMATION FOR SEQ ID NO:8:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: Synthetic oligonucleotide
   (xi) SEQUENCE DESCRIPTION: SEQ ID NO:8:
   CGTAGGGGAC AGCAAT                                                  16